# EUROPEAN PATENT APPLICATION

(11) **EP 4 740 974 A1**
(43) Date of publication of application: **13.05.2026**
(21) Application number: 24843074.6
(22) Date of filing: 12.07.2024
(51) Int. Cl.: A61L 33/06, A61L 27/16, A61L 27/34, A61L 27/40, A61L 27/50, A61L 29/08, A61L 29/14, A61L 31/10

(54) **MEDICAL INSTRUMENT AND MANUFACTURING METHOD THEREFOR**

(30) Priority: 14.07.2023 JP 2023116081
(71) Applicant: Terumo Kabushiki Kaisha, Tokyo 151-0072 (JP)
(72) Inventor: LIU, Yihua, Ashigarakami-gun, Kanagawa 259-0151 (JP); KITAHARA, Yoko, Ashigarakami-gun, Kanagawa 259-0151 (JP); HIRAKI, Akihiro, Ashigarakami-gun, Kanagawa 259-0151 (JP)
(74) Representative: Prüfer & Partner mbB Patentanwälte · Rechtsanwälte
(86) International application number: PCT/JP2024/025224
(87) International publication number: WO 2025/018283

(57) **Abstract**

Provided is a means configured to further improve antithrombogenicity in a medical device. A medical device of the present invention is a medical device including: a base material; an antithrombogenic layer including a polymer fixed to the base material, in which the polymer includes: a structural unit a derived from a first monomer bonded to the base material and including a reactive group that reacts with a hydroxyl group or a carboxyl group and an ethylenically unsaturated group; a structural unit B including a polymer chain including a structural unit b derived from a first antithrombogenic monomer including a carboxyl group and an ethylenically unsaturated group, and including a terminal of the polymer chain bonded to the structural unit a; and a structural unit C derived from a copolymer including a structural unit c1 derived from a second monomer including an amino group and bonded to the structural unit b and a structural unit c2 derived from a second antithrombogenic monomer.

## Description

### Technical Field

The present invention relates to a medical device and a method for manufacturing the same.

### Background Art

In recent years, medical materials utilizing various polymer materials or metal materials have been studied, and are expected to be used for membranes for artificial kidneys, membranes for plasma skimming, catheters, stents, membranes for artificial lungs, artificial blood vessels, anti-adhesion membranes, artificial skins, stent grafts, covered stents, guide wires, cardiac pacemakers, and the like. In these materials, a synthetic polymer material or metal material, which is a foreign substance for a living body, is used in contact with a biological tissue or a body fluid such as blood. Therefore, the medical materials are required to have biocompatibility. The biocompatibility required for a medical material varies depending on its purpose and use method, but a medical material used as a material in contact with blood is required to have characteristics (antithrombogenicity) of suppressing the blood coagulation system, suppressing the adhesion/activation of platelets, and suppressing the activation of the complement system.

Usually, the antithrombogenicity is imparted to the medical device by a method of coating a base material constituting the medical device with an antithrombogenic material or a method of fixing an antithrombogenic material on a surface of a base material. For example, Non Patent Literature 1 discloses a method for modifying medical stretched polyethylene tetrafluoroethylene (ePTFE) by surface graft polymerization. More specifically, this literature describes that a peroxide group is generated by subjecting an ePTFE surface to an argon plasma treatment, a methacryloyl group is constructed by reacting glycidyl methacrylate (GMA), and an MPC polymer chain is fixed to the ePTFE surface via a structural unit derived from GMA by graft polymerization of 2-methacryloyloxyethyl phosphorylcholine (MPC). According to this literature, graft polymerization using GMA is considered to provide a new method for modifying a chemically inert ePTFE surface to function as a new medical device.

### Citation List

### Non Patent Literature

Non Patent Literature 1: Yihua Liu et al., "A surface graft polymerization process on chemically stable medical ePTFE for suppressing platelet adhesion and activation", Biomaterials Science, The Royal Society of Chemistry, 07 June 2018, Issue 6, Page 1908 to 1915.

### Summary of Invention

However, according to the study of the present inventors, it has been found that there is a case where a medical device having sufficient antithrombogenicity cannot be obtained even by the method described in Non Patent Literature 1.

Therefore, an object of the present invention is to provide a means configured to further improve antithrombogenicity in a medical device.

The present inventors have intensively studied to solve the above problems. As a result, the present inventors have found that the above problems can be solved by graft polymerization of an antithrombogenic monomer on a surface of a base material via a predetermined structural unit to construct a polymer chain; and bonding a copolymer derived from an antithrombogenic monomer and the polymer chain, thereby completing the present invention.

That is, the above object can be achieved by the present invention having the following configuration, and the present invention includes the following aspects and forms.

An aspect of the present invention is
1. A medical device including:
   a base material;
   an antithrombogenic layer including a polymer fixed to the base material,
   in which the polymer includes:
      a structural unit a derived from a first monomer bonded to the base material and including a reactive group that reacts with a hydroxyl group or a carboxyl group and an ethylenically unsaturated group;
      a structural unit B including a polymer chain including a structural unit b derived from a first antithrombogenic monomer including a carboxyl group and an ethylenically unsaturated group, and including a terminal of the polymer chain bonded to the structural unit a; and
      a structural unit C derived from a copolymer including a structural unit c1 derived from a second monomer including an amino group and a structural unit c2 derived from a second antithrombogenic monomer, the structural unit C bonded to the structural unit b.
2. In the medical device described in the above 1., the second antithrombogenic monomer is preferably one or two or more selected from a monomer represented by the following Formula (I); 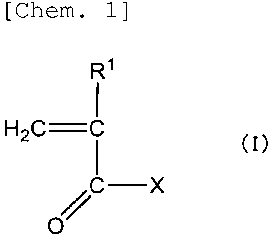 wherein,
   R¹ represents a hydrogen atom or a methyl group,
   X represents a group represented by formula: -O-(R²O)ₘ-R³, a group represented by formula: -O-(CH₂CHOH)ₙ-CH₂OH, or a tetrahydrofurfuryloxy group, in which each R² independently represents a linear or branched alkylene group having carbon atoms in a range of 1 to 4, R³ represents a linear or branched alkyl group having carbon atoms in a range of 1 to 4, m represents an integer in a range of 1 to 5, and n represents an integer in a range of 1 to 5.
3. In the medical device described in the above 1. or 2., the second antithrombogenic monomer is preferably one or two or more selected from methoxymethyl acrylate, 2-methoxyethyl acrylate (MEA), 3-methoxypropyl acrylate (MC3A), ethoxymethyl acrylate, ethoxyethyl acrylate (EEA), 2-(2-ethoxyethoxy)ethyl acrylate (EEEA), ethoxypropyl acrylate, ethoxybutyl acrylate, propoxymethyl acrylate, butoxyethyl acrylate, methoxybutyl acrylate, tetrahydrofurfuryl acrylate (THFA), methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, and glycerol (meth)acrylate.
4. In the medical device described in any one of the above 1. to 3., the first monomer is preferably one or two or more selected from glycidyl acrylate, glycidyl methacrylate (GMA), 3,4-epoxycyclohexyl methyl acrylate, 3,4-epoxycyclohexyl methyl methacrylate, β-methyl glycidyl acrylate, β-methyl glycidyl methacrylate, and allyl glycidyl ether.
5. In the medical device described in any one of the above 1. to 4., the first antithrombogenic monomer is preferably one or two or more selected from a monomer represented by the following Formula (II); 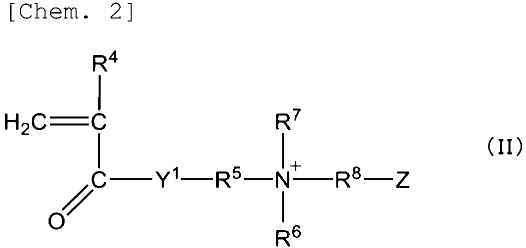 wherein,
   R⁴ represents a hydrogen atom or a methyl group,
   R⁵ represents a linear or branched alkylene group having carbon atoms in a range of 1 to 6,
   R⁶ and R⁷ each independently represent a linear or branched alkyl group having carbon atoms in a range of 1 to 4,
   R⁸ represents a linear or branched alkylene group having carbon atoms in a range of 1 to 4,
   Z represents -COO⁻, and
   Y¹ represents an oxygen atom or -NH-.
6. In the medical device described in any one of the above 1. to 5., the first antithrombogenic monomer is preferably one or two or more selected from N-(meth)acryloyloxymethyl-N,N-dimethylammonium-α-N-methylcarboxybetaine, N-(meth)acryloyloxyethyl-N,N-dimethylammonium-α-N-methylcarboxybetaine, N-(meth)acryloyloxypropyl-N,N-dimethylammonium-α-N-methylcarboxybetaine, N-(meth)acryloyloxymethyl-N,N-diethylammonium-α-N-methylcarboxybetaine, N-(meth)acryloyloxyethyl-N,N-diethylammonium-α-N-methylcarboxybetaine, and N-(meth)acryloyloxypropyl-N,N-diethylammonium-α-N-methylcarboxybetaine.
7. In the medical device described in any one of the above 1. to 6., the second monomer is preferably one or two or more selected from a monomer represented by following Formula (III) and a salt thereof; 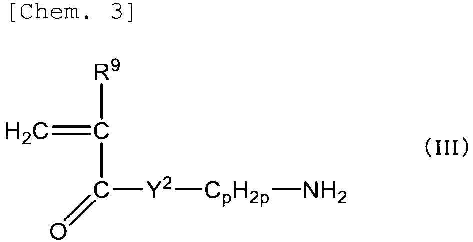 wherein,
   R⁹ represents a hydrogen atom or a methyl group,
   Y² represents an oxygen atom or -NH-, and
   p represents an integer in a range of 1 to 4.
8. In the medical device described in any one of the above 1. to 7., the second monomer is preferably one or two or more selected from aminomethyl acrylamide, aminomethyl methacrylamide, aminoethyl acrylamide, aminoethyl methacrylamide, aminopropyl acrylamide, aminopropyl methacrylamide (APMA), aminobutyl acrylamide, aminobutyl methacrylamide, aminomethyl acrylate, aminomethyl methacrylate, aminoethyl acrylate, aminoethyl methacrylate, aminopropyl acrylate, aminopropyl methacrylate, aminobutyl acrylate, aminobutyl methacrylate, and salts thereof.
9. In the medical device described in any one of the above 1. to 8., a molar ratio (c1 : c2) of the structural unit c1 to the structural unit c2 in the structural unit C is preferably in a range of 1 : 1 to 1 : 20.
10. In the medical device described in any one of the above 1. to 9., the base material preferably includes a plastic base material.
11. In the medical device described in the above 10, the plastic base material is preferably a porous body.
12. In the medical device described in any one of the above 1. to 11., the medical device is preferably an artificial blood vessel, a stent graft, a covered stent, or a catheter.

Another aspect of the present invention is
13. A method for manufacturing the medical device described in any one of 1. to 12., the manufacturing method including:
irradiating at least a part of a surface of the base material with plasma;
bringing the first monomer into contact with the base material after the plasma irradiation;
bringing the second monomer into contact with the base material after the first monomer is brought into contact; and
bringing the copolymer into contact with the base material after the second monomer is brought into contact.

### Brief Description of Drawings

Fig. 1 is a view for describing fixation of an antithrombogenic layer in the present invention.
Fig. 2 is a partial cross-sectional view schematically illustrating a structure of a representative embodiment of a medical device according to the present invention. In Fig. 2, reference numeral 10 denotes a base material, reference numeral 11 denotes an antithrombogenic layer, and reference numeral 100 denotes a medical device.
Fig. 3 is a partial cross-sectional view schematically illustrating a configuration example including a different structure as an application example of the embodiment of Fig. 2. In Fig. 3, reference numeral 10 denotes a base material, reference numeral 10a denotes a base material core portion, reference numeral 10b denotes a base material surface layer, reference numeral 11 denotes an antithrombogenic layer, and reference numeral 100 denotes a medical device.
Fig. 4 is a photograph showing a blood circulation circuit for a stent graft.
Fig. 5 is an appearance photograph of a stent graft base material made of PET (SG made of PET (plasma-untreated)) of Comparative Example 1 after a blood circulation test.
Fig. 6 is an appearance photograph of a stent graft made of PET in which an MEA-APMA copolymer is fixed via GMA and CBA polymer chains (SG-GMA-pCBA-p(MEA-APMA) made of PET) of Example 1 after a blood circulation test.
Fig. 7 is an SEM image obtained by observing, with a scanning electron microscope, an artificial blood vessel made of ePTFE (ePTFEg (plasma-untreated)) of Comparative Example 2 after a blood circulation test.
Fig. 8 is an SEM image obtained by observing, with a scanning electron microscope, an artificial blood vessel made of ePTFE and polymerized with CBA via GMA (ePTFEg-GMA-pCBA) of Comparative Example 3 after a blood circulation test.
Fig. 9 is an SEM image obtained by observing, with a scanning electron microscope, an artificial blood vessel made of ePTFE in which an MEA-APMA copolymer is fixed via GMA and CBA polymer chains (ePTFEg-GMA-pCBA-p(MEA-APMA)) of Example 2 after a blood circulation test.
Fig. 10 is an appearance photograph of a silicone tube (silicone tube (plasma-untreated)) of Comparative Example 4 after a blood circulation test.
Fig. 11 is an appearance photograph of a silicone tube in which an MEA-APMA copolymer is fixed via GMA and CBA polymer chains (silicone tube-GMA-pCBA-p(MEA-APMA)) of Example 3 after a blood circulation test.
Fig. 12 is an appearance photograph of a polyurethane tube in which an MEA-APMA copolymer is fixed via GMA and CBA polymer chains (PU tube-GMA-pCBA-p(MEA-APMA)) of Example 4 after a blood circulation test.

### Description of Embodiments

According to an aspect of the present invention, there is provided a medical device including a base material and an antithrombogenic layer including a polymer fixed to the base material. The polymer includes: a structural unit a derived from a first monomer bonded to the base material and including a reactive group that reacts with a hydroxyl group or a carboxyl group and an ethylenically unsaturated group; a structural unit B including a polymer chain including a structural unit b derived from a first antithrombogenic monomer including a carboxyl group and an ethylenically unsaturated group, and including a terminal of the polymer chain bonded to the structural unit a; and a structural unit C derived from a copolymer including a structural unit c1 derived from a second monomer including an amino group and a structural unit c2 derived from a second antithrombogenic monomer, the structural unit C bonded to the structural unit b.

The present inventors have surprisingly found that a medical device including such a configuration has excellent antithrombogenicity (that is, adhesion of proteins, cells, and the like that may constitute thrombus can be effectively suppressed). Here, the mechanism of exerting the above-described action and effect by the configuration of the present invention is presumed as follows.

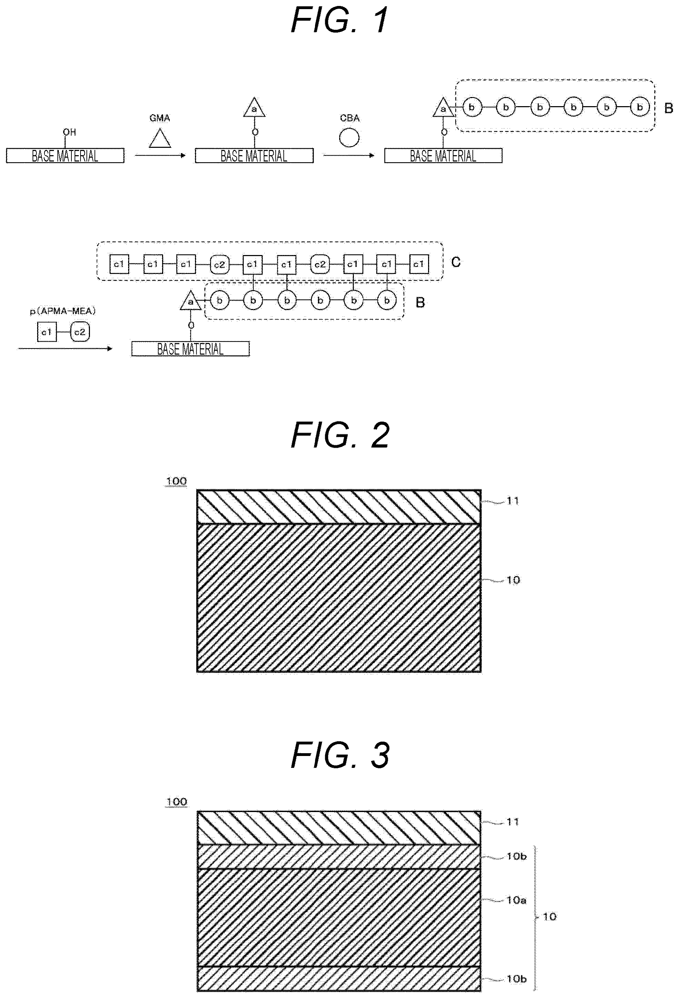

Fig. 1 is a view for describing fixation of an antithrombogenic layer in the present invention. As illustrated in Fig. 1, in the medical device according to the present invention, a first monomer (for example, glycidyl methacrylate (GMA)) including a reactive group (for example, a glycidyl group) that reacts with a hydroxyl group or a carboxyl group and an ethylenically unsaturated group is reacted with a base material including a hydroxyl group (-OH) and a carboxyl group (-COOH, not illustrated) formed on a surface thereof by plasma irradiation, and a structural unit a derived from the first monomer is fixed to a surface of the base material. As a result, the ethylenically unsaturated group is established on the surface of the base material. Thereafter, a first antithrombogenic monomer (for example, N-methacryloyloxyethyl-N,N-dimethylammonium-α-N-methylcarboxybetaine (CBA)) including a carboxyl group and an ethylenically unsaturated group is graft-polymerized with the ethylenically unsaturated group in the structural unit a to form a structural unit B including a polymer chain including a structural unit b derived from the first antithrombogenic monomer. Since the polymer chain is extended from the ethylenically unsaturated group of the structural unit a by graft polymerization, the structural unit a and the terminal of the polymer chain are bonded to each other. Thereafter, a copolymer (p(MEA-APMA)) including a structural unit c1 derived from a second monomer (for example, aminopropyl methacrylamide (APMA)) including an amino group (-NH₂) and a structural unit c2 derived from a second antithrombogenic monomer (for example, methoxyethyl acrylate (MEA)) is subjected to reaction (dehydration condensation reaction) with the polymer chain. Thus, the carboxyl group derived from CBA and the amino group derived from APMA form
an amide bond (-C(=O)-NH-), and the structural unit C derived from the copolymer is fixed. As a result, the antithrombogenic layer including a polymer including the structural unit a, the structural unit B, and the structural unit C is fixed to the surface of the base material via a covalent bond. With such a configuration, as compared with Non Patent Literature 1, more polymer derived from an antithrombogenic monomer can be fixed to the surface of the base material, and thus the antithrombogenicity in the medical device can be further improved. Note that, in the technique described in Non Patent Literature 1, a polymer derived from an antithrombogenic monomer is fixed to the surface of the base material only by graft polymerization. According to the study of the present inventors, it has been found that there is a limit to the extension of the polymer chain by graft polymerization, and thus the technique described in Non Patent Literature 1 cannot impart sufficient antithrombogenicity in some cases. Since the antithrombogenic layer according to the present invention also includes the structural unit C derived from a copolymer including the structural unit c2 derived from the second antithrombogenic monomer in addition to the structural unit B including a polymer chain including the structural unit b derived from the first antithrombogenic monomer, the antithrombogenicity of the medical device can be further improved. Therefore, according to the present invention, there is provided a means configured to further improve antithrombogenicity in a medical device.

Note that the above mechanism is an inference and does not limit the technical scope of the present invention at all.

In the present specification, the medical device including the above configuration is also simply referred to as "medical device according to the present invention" or "medical device". In the present specification, the "structural unit a derived from a first monomer including a reactive group that reacts with a hydroxyl group or a carboxyl group and an ethylenically unsaturated group" is also simply referred to as "structural unit a according to the present invention" or "structural unit a". In the present specification, the "structural unit b derived from a first antithrombogenic monomer including a carboxyl group and an ethylenically unsaturated group" is also simply referred to as "structural unit b according to the present invention" or "structural unit b". In the present specification, the "polymer chain including a structural unit b derived from a first antithrombogenic monomer including a carboxyl group and an ethylenically unsaturated group" is also simply referred to as "polymer chain according to the present invention" or "polymer chain". In the present specification, the "structural unit B including a polymer chain including a structural unit b derived from a first antithrombogenic monomer including a carboxyl group and an ethylenically unsaturated group, and including a terminal of the polymer chain bonded to the structural unit a" is also simply referred to as "structural unit B according to the present invention" or "structural unit B". In the present specification, the "structural unit c1 derived from a second monomer including an amino group" is also simply referred to as "structural unit c1 according to the present invention" or "structural unit c1". In the present specification, the "structural unit c2 derived from a second antithrombogenic monomer" is also simply referred to as "structural unit c2 according to the present invention" or "structural unit c2". In the present specification, the "copolymer including a structural unit c1 derived from a second monomer including an amino group and bonded to the structural unit b and a structural unit c2 derived from a second antithrombogenic monomer" is also simply referred to as "copolymer according to the present invention" or "copolymer". In the present specification, the "structural unit C derived from a copolymer including a structural unit c1 derived from a second monomer including an amino group and bonded to the structural unit b and a structural unit c2 derived from a second antithrombogenic monomer" is also simply referred to as "structural unit C according to the present invention" or "structural unit C". In the present specification, the polymer including the structural unit a, the structural unit B, and the structural unit C is also simply referred to as "polymer according to the present invention" or "polymer".

In the present specification, when a structural unit is defined to be "derived" from a certain monomer, it means that the structural unit is a structural unit generated by a condensation reaction of a reactive group of the corresponding monomer and/or generated by cleavage of one bond of an ethylenically unsaturated group (polymerizable unsaturated double bond).

In the present specification, the term "(meth)acryl" encompasses both acryl and methacryl. Therefore, for example, the term "(meth)acrylic acid" encompasses both acrylic acid and methacrylic acid. Similarly, the term "(meth)acryloyl" encompasses both acryloyl and methacryloyl. Therefore, for example, the term "(meth)acryloyl group" encompasses both an acryloyl group and a methacryloyl group. Further similarly, the term "(meth)acrylate" encompasses both acrylates and methacrylates. Thus, for example, the term "alkoxyalkyl (meth)acrylate" encompasses both an alkoxyalkyl acrylate and an alkoxyalkyl methacrylate.

In the present specification, a range from "X to Y" includes X and Y, and indicates "X or more and Y or less". Furthermore, the expression "A and/or B" represents either A or B and also all combinations of A and B, and specifically, the expression represents at least one of A and B, that is, A, B, and combinations of A and B. Unless otherwise specified, operations and measurements of physical properties and the like are performed at room temperature (20 to 25°C) and at relative humidity of 40 to 50% RH.

Hereinafter, embodiments of the present invention will be described. Note that the present invention is not limited only to the following embodiments, and various modifications can be made within the scope of claims. The embodiments described in the present specification can be arbitrarily combined with each other to form another embodiment. The dimensional ratios in the drawings are exaggerated for convenience of description and may be different from actual ratios. In a case where embodiments of the present invention are described with reference to the drawings, the same elements are denoted by the same reference numerals in the description of the drawings, and redundant description will be omitted.

Throughout the present specification, expression of a singular should be understood to include also a concept of a plural thereof unless otherwise stated. Thus, an article of a singular (for example, "a", "an", and "the" in English) should be understood to include also a concept of a plural thereof unless otherwise stated. Terms used in the present specification should be understood to be used in a sense commonly used in the art unless otherwise stated. Therefore, unless otherwise defined, all technical and scientific terms used in the present specification have the same meanings as commonly understood by a person skilled in the art to which the present invention belongs. In the case of contradiction, priority is given to the present specification (including definitions).

### [Medical device]

Hereinafter, a configuration of the medical device according to an embodiment of the present invention will be described with reference to the accompanying drawings.

Fig. 2 is a partial cross-sectional view schematically illustrating a structure of a representative embodiment of a medical device according to the present invention. Fig. 3 is a partial cross-sectional view schematically illustrating a configuration example including a different structure as an application example of the present embodiment.

As illustrated in Figs. 2 and 3, a medical device 100 according to an embodiment of the present invention includes a base material 10 and an antithrombogenic layer 11 formed on at least a part of the base material 10. Here, in the medical device 100 of the present embodiment, the base material 10 and the antithrombogenic layer 11 are directly laminated in this order. That is, the base material 10 and the antithrombogenic layer 11 are adjacently laminated in this order.

In the present embodiment, the polymer constituting the antithrombogenic layer is chemically bonded to the surface of the base material by a covalent bond (more specifically, the reactive group in the first monomer and a hydroxyl group (-OH) or a carboxyl group (-COOH) generated by the plasma treatment are bonded to each other by a covalent bond), whereby the antithrombogenicity can be maintained for a long period of time.

In the present specification, the expression "the antithrombogenic layer is formed on at least a part of the base material" is used because in an artificial blood vessel, a stent graft, a covered stent, a catheter, and the like, which are examples of use application of the medical device, it is not always necessary to impart antithrombogenicity to all surfaces (entire surface) of these medical devices, and it is sufficient that the antithrombogenic layer is formed only on a surface portion (which may be a part or all) required to have antithrombogenicity. Therefore, the antithrombogenic layer is preferably formed on at least a portion of the base material 10 that is in contact with blood. For example, when the medical device is an artificial blood vessel, a stent graft, a covered stent, or a catheter, the antithrombogenic layer is preferably formed on the entire surface of the base material.

### <<Base material (base material layer)>>

The base material 10 constituting the medical device according to an embodiment of the present invention is not particularly limited, but preferably includes at least one of a plastic base material (polymer base material) and a metal base material. As the plastic base material, an optimum base material can be appropriately selected according to characteristics (for example, a mechanical strength and the like) required for medical devices such as an artificial blood vessel, a stent graft, a covered stent, and a catheter, and as an example, a plastic base material having characteristics required for the base material such as a mechanical strength, flexibility, and smoothness is preferable. As the metal base material, an optimum base material can be appropriately selected according to characteristics (for example, a mechanical strength and the like) required for medical devices such as a stent graft, a covered stent, a stent, a guide wire, and a cardiac pacemaker. As an example, a metal base material having characteristics required for the base material such as a mechanical strength, elasticity, and toughness is preferable.

It is preferable that at least a surface of the base material 10 is comprised of a plastic base material or a metal base material. As an example, as illustrated in Fig. 2, the base material may be entirely (fully) comprised (formed) of a plastic base material or a metal base material. As illustrated in Fig. 3, the base material may include a structure in which a base material surface layer 10b formed of a plastic base material or a metal base material may be configured (formed) on a surface of a base material core portion 10a formed of a material such as a ceramic material. The base material core portion 10a and the base material surface layer 10b may be formed into a composite (subjected to an appropriate reaction treatment) to form the base material 10. Accordingly, the base material core portion 10a may be a multilayer structure obtained by laminating different materials in multiple layers, or a structure (for example, a composite) obtained by connecting members formed from different materials for each part of the medical device, or the like. Another middle layer (not illustrated) may be further formed between the base material core portion 10a and the base material surface layer 10b. The base material surface layer 10b may also be a multilayer structure obtained by laminating different materials in multiple layers, a structure (composite) obtained by connecting members formed from different materials for each part of the medical device, or the like, as long as the surface of the base material surface layer is comprised (formed) of a plastic base material or a metal base material.

The plastic material (polymer material) constituting (forming) the base material 10 and the base material surface layer 10b is not particularly limited, and a polymer material generally used for medical devices such as an artificial blood vessel, a stent graft, a covered stent, and a catheter is used. Specific examples thereof include polyamide resins such as nylon 6, nylon 11, nylon 12, and nylon 66 (all are registered trademarks), polyethylene such as linear low-density polyethylene (LLDPE), low-density polyethylene (LDPE), high-density polyethylene (HDPE), and modified polyethylene, polyolefin resins such as polypropylene, polyester resins such as polyethylene terephthalate (PET), polytrimethylene terephthalate (PTT), polybutylene terephthalate (PBT), and polyethylene naphthalate (PEN), styrene resins such as polystyrene, cyclic polyolefin resins, modified polyolefin resins, epoxy resins, urethane resins, diallyl phthalate resins (allyl resins), polycarbonate resins, fluororesins such as polytetrafluoroethylene (PTFE), ePTFE (stretched polyethylene tetrafluoroethylene: expanded polytetrafluoroethylene), polyurethane resins, amino resins (urea resins, melamine resins, benzoguanamine resins), acrylic resins, polyacetal resins, vinyl acetate resins, phenol resins, vinyl chloride resins, silicone resins, polyether resins such as polyether ether ketone (PEEK), and polyimide resins. These may be used singly or in combination of two or more kinds thereof. The polymer material can be appropriately selected according to the use application of the medical device. The polymer material (plastic) constituting (forming) a base material of an artificial blood vessel, a stent graft, a covered stent, a catheter, or the like, which is an example of use application preferably includes one or two or more selected from the group consisting of a polyester resin, a fluororesin, a polyurethane resin, and a silicone resin, and is more preferably one or two or more selected from the group consisting of a polyester resin, a fluororesin, a polyurethane resin, and a silicone resin. From the viewpoint of a mechanical strength, the polymer material (plastic) more preferably includes a polyester resin.

The metal material constituting (forming) the base material 10 and the base material surface layer 10b is not particularly limited, and a metal material generally used for medical devices such as a stent graft, a covered stent, a stent, a guide wire, and a cardiac pacemaker is used. Specific examples thereof include gold, platinum, silver, copper, nickel, cobalt, titanium, iron, aluminum, tin, stainless steel (for example, SUS304, SUS314, SUS316, SUS316L, SUS420J2, SUS630, SUS631), a nickel-titanium alloy, a nickel-cobalt alloy, a cobalt-chromium alloy, and a zinc-tungsten alloy. These may be used singly or in combination of two or more kinds thereof. The metal material can be appropriately selected according to the use application of the medical device. The metal material constituting (forming) a base material of a stent graft, a covered stent, a stent, a guide wire, a cardiac pacemaker, or the like, which is an example of use application is more preferably one or two or more selected from the group consisting of stainless steel and a nickel-titanium alloy, and is still more preferably one or two or more selected from the group consisting of a nickel-titanium alloy from the viewpoint of versatility. That is, in the medical device according to an embodiment of the present invention, the metal is one or two or more selected from the group consisting of gold, platinum, silver, copper, nickel, cobalt, titanium, iron, aluminum, tin, stainless steel, a nickel-titanium alloy, a nickel-cobalt alloy, a cobalt-chromium alloy, and a zinc-tungsten alloy. According to a more preferred aspect, the metal is one or two or more selected from the group consisting of stainless steel and a nickel-titanium alloy. According to a still more preferred aspect, the metal is one or two or more selected from the group consisting of a nickel-titanium alloy.

The material that can be used for the above-described base material core portion 10a is not particularly limited, and a material that can sufficiently exhibit an optimum function as the base material core portion 10a may be appropriately selected according to the application of the medical device. When the base material 10 and the base material surface layer 10b are comprised of a plastic material, examples of the material of the base material core portion 10a include the above-described various metal materials, inorganic materials such as various ceramic materials, and a metal-ceramic composite, but are not limited thereto at all. When the base material 10 and the base material surface layer 10b are comprised of a metal material, examples of the material of the base material core portion 10a include the above-described various plastic materials and inorganic materials such as various ceramic materials, but are not limited thereto at all.

Alternatively, the base material core portion 10a may be comprised of a plastic material or a metal material other than the plastic material or the metal material forming (constituting) the base material surface layer 10b. Examples of such a plastic material or metal material are not particularly limited, and include the same materials as described above. These may be used singly or in combination of two or more kinds thereof.

Preferably, the base material 10 includes a plastic base material or is comprised of a plastic base material, or the base material surface layer 10b includes a plastic base material or is comprised of a plastic base material. More preferably, the base material 10 is comprised of a plastic material, or the base material surface layer 10b is comprised of a plastic material. That is, according to an aspect of the present invention, the base material includes a plastic base material. According to a more preferred aspect of the present invention, the base material includes a plastic base material.

The shape of the base material 10 is not particularly limited, and is appropriately selected from a sheet shape, a film shape, a tubular shape, a shape having a plurality of branch pipes (for example, Y shape), a linear shape (wire), a fiber (yarn) shape, and the like according to a use mode. The base material 10 is preferably composed of a porous body. In the present specification, the "porous body" in the present invention means a structure with voids that allow the progress or invasion of body fluids and/or cells. The average pore diameter of the pores is preferably in a range of 1 to 100 µm, and more preferably in a range of 1 to 20 µm. Here, the average pore diameter is measured by the following method. First, the surface of the base material is observed using a scanning electron microscope (SEM), and the pore area of each pore observed in several to several tens of fields of view is measured. Then, the diameter (equivalent circle diameter) of a perfect circle having the same area as the pore area of each pore is calculated, and the arithmetic average value thereof is taken as the average pore diameter. Since the porous body including pores having the above pore diameter has favorable cell adhesion to vascular endothelial cells, the surface of the metal base material exposed in blood is easily covered with endothelial cells, and the risk of thrombus formation can be further reduced. That is, in the medical device according to an embodiment of the present invention, the base material is a porous body. According to a more preferred embodiment of the present invention, the base material includes a plastic base material, and the plastic base material is a porous body. Note that the porous body formed of the plastic base material is not particularly limited, and examples thereof include a cloth comprised of fibers (yarns) (woven fabric, nonwoven fabric), and ePTFE (stretched polyethylene tetrafluoroethylene: expanded polytetrafluoroethylene).

### <<Antithrombogenic layer>>

The antithrombogenic layer according to the present invention includes a polymer fixed to the base material. The polymer includes: a structural unit a derived from a first monomer bonded to the base material and including a reactive group that reacts with a hydroxyl group or a carboxyl group and an ethylenically unsaturated group; a structural unit B including a polymer chain including a structural unit b derived from a first antithrombogenic monomer including a carboxyl group and an ethylenically unsaturated group, and including a terminal of the polymer chain bonded to the structural unit a; and a structural unit C derived from a copolymer including a structural unit c1 derived from a second monomer including an amino group and a structural unit c2 derived from a second antithrombogenic monomer, the structural unit C bonded to the structural unit b. The antithrombogenic layer may be in a one-layer form or in a laminated form of two or more layers. Preferably, the antithrombogenic layer is in a one-layer form. Note that, in the present specification, the "antithrombogenicity" refers to a property of reducing blood coagulation on a surface in contact with blood.

The first monomer is not particularly limited as long as it includes a reactive group that reacts with a hydroxyl group or a carboxyl group and an ethylenically unsaturated group.

Examples of the reactive group that reacts with a hydroxyl group or a carboxyl group in the first monomer include an epoxy group (-C₂H₃O), a glycidyl group (-CH₂(C₂H₃O)), an amino group (-NH₂). Among them, the reactive group is preferably an epoxy group or a glycidyl group, and more preferably a glycidyl group because of having high reactivity. More specifically, when the plastic base material is subjected to a plasma treatment, a hydroxyl group (-OH) and a carboxyl group (-COOH) are formed on the surface of the base material. When the metal base material is subjected to a plasma treatment, a hydroxyl group (-OH) is formed on the surface of the base material. The reactive group is subjected to a reaction (condensation reaction) with a hydroxyl group (-OH) and a carboxyl group (-COOH) to form a covalent bond (for example, an ether bond (-O-), an amide bond (-C(=O)-NH-), or the like). As a result, the antithrombogenic layer is firmly fixed to the base material.

The ethylenically unsaturated group in the first monomer is a group that may be subjected to polymerization reaction with a first antithrombogenic monomer described later. Examples of the ethylenically unsaturated group include an acryloyl group (CH₂=CH-C(=O)-), a methacryloyl group (CH₂=C(CH₃)-C(=O)-), a vinyl group (CH₂=CH-), and an allyl group (CH₂=CH-CH₂-). Among them, the first monomer preferably includes at least one of an acryloyl group and a methacryloyl group.

In an embodiment, specific examples of the first monomer include, but are not limited to, (meth)acrylic acid esters having a glycidyl group (epoxy group), such as glycidyl acrylate, glycidyl methacrylate (GMA), 3,4-epoxycyclohexylmethyl acrylate, 3,4-epoxycyclohexylmethyl methacrylate, β-methylglycidyl acrylate, or β-methylglycidyl methacrylate; and a vinyl ether having a glycidyl group (epoxy group), such as allyl glycidyl ether. These may be used singly or in combination of two or more kinds thereof. Among them, from a viewpoint of further improving reactivity with a hydroxyl group or a carboxyl group, the first monomer preferably includes one or two or more selected from the group consisting of glycidyl acrylate, glycidyl methacrylate, 3,4-epoxycyclohexylmethyl acrylate, 3,4-epoxycyclohexylmethyl methacrylate, β-methylglycidyl acrylate, and β-methylglycidyl methacrylate. The first monomer more preferably includes one or two selected from the group consisting of glycidyl acrylate and glycidyl methacrylate. The first monomer still more preferably includes glycidyl acrylate or glycidyl methacrylate, particularly preferably includes glycidyl methacrylate, and is most preferably glycidyl methacrylate. That is, in a most preferred aspect of the present invention, the structural unit a is derived from glycidyl methacrylate (GMA).

In another embodiment, specific examples of the first monomer include, but are not limited to, aminomethyl acrylamide, aminomethyl methacrylamide, aminoethyl acrylamide, aminoethyl methacrylamide, aminopropyl acrylamide, aminopropyl methacrylamide (APMA), aminobutyl acrylamide, and aminobutyl methacrylamide. These may be used singly or in combination of two or more kinds thereof. Among them, from a viewpoint of further improving reactivity with a hydroxyl group or a carboxyl group, the first monomer preferably includes aminopropyl methacrylamide (APMA).

The first antithrombogenic monomer is not particularly limited as long as it includes a carboxyl group and an ethylenically unsaturated group, and the homopolymer thereof has antithrombogenicity.

Specific examples of the first antithrombogenic monomer include a monomer represented by the following Formula (II). Homopolymers derived from these monomers may exhibit excellent antithrombogenicity.

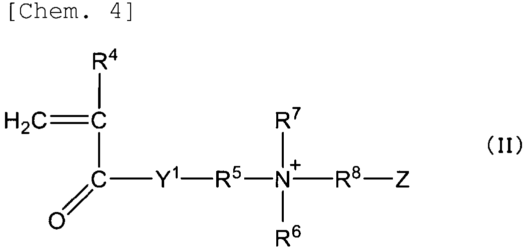

In the above Formula (II),
R⁴ represents a hydrogen atom or a methyl group. From the viewpoint of obtaining excellent antithrombogenicity, R⁴ is preferably a methyl group.

R⁵ represents a linear or branched alkylene group having carbon atoms in a range of 1 to 6. Specific examples of the linear or branched alkylene group having carbon atoms in a range of 1 to 6 include a methylene group, an ethylene group, a trimethylene group, a propylene group, a tetramethylene group, a pentamethylene group, and a hexamethylene group. Among them, from the viewpoint of obtaining excellent antithrombogenicity, R⁵ is preferably a linear or branched alkylene group having carbon atoms in a range of 1 to 4, more preferably a methylene group, an ethylene group, or a trimethylene group, and particularly preferably an ethylene group or a trimethylene group.

R⁶ and R⁷ each independently represent linear or branched alkyl group having carbon atoms in a range of 1 to 4. Specific examples of the linear or branched alkyl group having carbon atoms in a range of 1 to 4 include a methyl group, an ethyl group, a propyl group, an isopropyl group, a butyl group, an isobutyl group, a sec-butyl group, and a tert-butyl group. Among them, from the viewpoint of obtaining excellent antithrombogenicity, R⁶ and R⁷ are each independently preferably a linear or branched alkyl group having carbon atoms in a range of 1 to 3, more preferably an alkyl group having one or two carbon atoms (methyl group, ethyl group), and particularly preferably a methyl group.

R⁸ represents a linear or branched alkylene group having carbon atoms in a range of 1 to 4. Specific examples of the linear or branched alkylene group having carbon atoms in a range of 1 to 4 include a methylene group, an ethylene group, a trimethylene group, a propylene group, or a tetramethylene group. Among them, from the viewpoint of obtaining excellent antithrombogenicity, R⁸ is preferably a methylene group, an ethylene group, or a trimethylene group, and more preferably a methylene group.

Z represents -COO⁻.

Y¹ represents an oxygen atom or -NH-. From the viewpoint of obtaining excellent antithrombogenicity, Y¹ is preferably an oxygen atom.

Examples of the carboxybetaine-type zwitterionic monomer represented by the above Formula (II) include N-(meth)acryloyloxymethyl-N,N-dimethylammonium-α-N-methylcarboxybetaine, N-(meth)acryloyloxyethyl-N,N-dimethylammonium-α-N-methylcarboxybetaine, N-(meth)acryloyloxypropyl-N,N-dimethylammonium-α-N-methylcarboxybetaine, N-(meth)acryloyloxymethyl-N,N-diethylammonium-α-N-methylcarboxybetaine, N-(meth)acryloyloxyethyl-N,N-diethylammonium-α-N-methylcarboxybetaine, and N-(meth)acryloyloxypropyl-N,N-diethylammonium-α-N-methylcarboxybetaine. These may be used singly or in combination of two or more kinds thereof. Note that, in the latter case, the structural unit derived from each monomer may exist in a block shape or in a random shape. Among them, from the viewpoint of obtaining excellent antithrombogenicity, N-(meth)acryloyloxyethyl-N,N-dimethylammonium-α-N-methylcarboxybetaine is preferable, and N-methacryloyloxyethyl-N,N-dimethylammonium-α-N-methylcarboxybetaine (CBA) is more preferable. That is, in a most preferred aspect of the present invention, the structural unit b is derived from N-methacryloyloxyethyl-N,N-dimethylammonium-α-N-methylcarboxybetaine (CBA).

The second monomer is not particularly limited as long as it includes an amino group. The amino group may form an amide bond with a carboxyl group derived from the first antithrombogenic monomer. Note that the homopolymer of the second monomer may have antithrombogenicity.

Specific examples of the second monomer include a monomer represented by the following Formula (III) and a salt thereof.

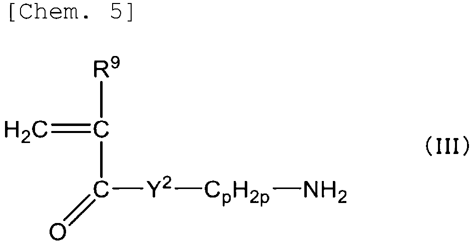

In the above Formula (III),
R⁹ represents a hydrogen atom or a methyl group. From the viewpoint of obtaining excellent antithrombogenicity, R² is preferably a methyl group.

Y² represents an oxygen atom or -NH-. From the viewpoint of obtaining excellent antithrombogenicity, Y² is preferably -NH-.

p represents an integer in a range of 1 to 4. From the viewpoint of obtaining excellent antithrombogenicity, p is preferably an integer in a range of 2 to 4, more preferably 2 or 3, and still more preferably 3.

Specific examples of the second monomer represented by the above Formula (III) include, but are not limited to, aminomethyl acrylamide, aminomethyl methacrylamide, aminoethyl acrylamide, aminoethyl methacrylamide, aminopropyl acrylamide, aminopropyl methacrylamide (APMA), aminobutyl acrylamide, aminobutyl methacrylamide, aminomethyl acrylate, aminomethyl methacrylate, aminoethyl acrylate, aminoethyl methacrylate, aminopropyl acrylate, aminopropyl methacrylate, aminobutyl acrylate, and aminobutyl methacrylate. These may be used singly or in combination of two or more kinds thereof. Note that, in the latter case, the structural unit derived from each monomer may exist in a block shape or in a random shape. Among them, from the viewpoint that reactivity with a carboxyl group can be further improved and antithrombogenicity can be further imparted, the second monomer is preferably one or two or more selected from aminomethyl acrylamide, aminomethyl methacrylamide, aminoethyl acrylamide, aminoethyl methacrylamide, aminopropyl acrylamide, aminopropyl methacrylamide (APMA), aminobutyl acrylamide, aminobutyl methacrylamide, aminomethyl acrylate, aminomethyl methacrylate, aminoethyl acrylate, aminoethyl methacrylate, aminopropyl acrylate, aminopropyl methacrylate, aminobutyl acrylate, aminobutyl methacrylate, and salts thereof. The second monomer is more preferably one or two or more selected from aminomethyl acrylamide, aminomethyl methacrylamide, aminoethyl acrylamide, aminoethyl methacrylamide, aminopropyl acrylamide, aminopropyl methacrylamide (APMA), aminobutyl acrylamide, aminobutyl methacrylamide, and salts thereof. The second monomer is still more preferably one or two or more selected from aminoethyl acrylamide, aminoethyl methacrylamide, aminopropyl acrylamide, aminopropyl methacrylamide (APMA), and salts thereof. The second monomer is particularly preferably one or two or more selected from aminopropyl acrylamide, aminopropyl methacrylamide (APMA), and salts thereof. The second monomer is most preferably selected from aminopropyl methacrylamide (APMA) and a salt thereof. That is, in a most preferred aspect of the present invention, the structural unit c1 is derived from aminopropyl methacrylamide (APMA).

The second antithrombogenic monomer is not particularly limited as long as the homopolymer thereof has antithrombogenicity.

Specific examples of the second antithrombogenic monomer include a monomer represented by the following Formula (I). Homopolymers derived from these monomers have cell adhesion to vascular endothelial cells in addition to excellent antithrombogenicity. From this point, by using one or two or more selected from the monomer represented by the above Formula (I) as the second antithrombogenic monomer, an antithrombogenic layer having both antithrombogenicity and vascular endothelial cell adhesion can be formed on the surface of the base material. As a result, for example, in a medical device such as an artificial blood vessel, a stent graft, a covered stent, or a catheter, the surface of the base material exposed in blood is easily covered with endothelial cells, and the risk of thrombus formation can be further reduced.

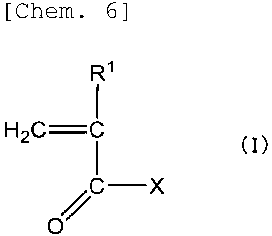

In the above Formula (I),
R¹ represents a hydrogen atom or a methyl group. From the viewpoint of obtaining excellent antithrombogenicity, R¹ is preferably a hydrogen atom.

X represents a group represented by formula: -O-(R²O)ₘ-R³, a group represented by the formula: -O-(CH₂CHOH)ₙ-CH₂OH, or a tetrahydrofurfuryloxy group. From the viewpoint of obtaining excellent antithrombogenicity, X is preferably a group represented by formula: -O-(R²O)ₘ-R³.

When X is a group represented by formula: -O-(R²O)ₘ-R³, each R² independently represents a linear or branched alkylene group having carbon atoms in a range of 1 to 4, and specific examples thereof include a methylene group, an ethylene group, a trimethylene group, a propylene group, and a tetramethylene group. Among them, from the viewpoint of obtaining excellent antithrombogenicity, each R² is independently more preferably a methylene group, an ethylene group, or a trimethylene group, particularly preferably an ethylene group or a trimethylene group, and most preferably an ethylene group. Note that, when there are a plurality of -R²O- (m is 2 or more), each -R²O- may be the same or different. R³ is a linear or branched alkyl group having carbon atoms in a range of 1 to 4, and specific examples thereof include linear or branched alkyl groups such as a methyl group, an ethyl group, a propyl group, an isopropyl group, a butyl group, an isobutyl group, a sec-butyl group, and a tert-butyl group. Among them, from the viewpoint of obtaining excellent antithrombogenicity, R³ is preferably a linear or branched alkyl group having carbon atoms in a range of 1 to 3, more preferably an alkyl group having one or two carbon atoms (methyl group, ethyl group), and particularly preferably a methyl group. m represents the number of (-R²O-) groups and is an integer in a range of 1 to 5, and from the viewpoint of obtaining excellent antithrombogenicity, m is preferably an integer in a range of 1 to 3, more preferably 1 or 2, and particularly preferably 1.

When X is a group represented by formula: -O-(CH₂CHOH)ₙ-CH₂OH, n represents the number of (-CH₂CHOH-) groups and is an integer in a range of 1 to 5. From the viewpoint of improving antithrombogenicity, n is preferably an integer in a range of 1 to 3, more preferably 1 or 2, and particularly preferably 1.

Specific examples of the monomer represented by the above Formula (I) include, but are not limited to, methoxymethyl acrylate, 2-methoxyethyl acrylate (MEA), 3-methoxypropyl acrylate (MC3A), ethoxymethyl acrylate, ethoxyethyl acrylate (EEA), 2-(2-ethoxyethoxy)ethyl acrylate (EEEA), ethoxypropyl acrylate, ethoxybutyl acrylate, propoxymethyl acrylate, butoxyethyl acrylate, methoxybutyl acrylate, tetrahydrofurfuryl acrylate (THFA), methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, and glycerol (meth)acrylate. These may be used singly or in combination of two or more kinds thereof. Note that, in the latter case, the structural unit derived from each monomer may exist in a block shape or in a random shape. Among them, 2-methoxyethyl acrylate (MEA), 3-methoxypropyl acrylate (MC3A), and tetrahydrofurfuryl acrylate (THFA) are preferable, and 2-methoxyethyl acrylate (MEA) and 3-methoxypropyl acrylate (MC3A) are more preferable. 2-Methoxyethyl acrylate (MEA) is still more preferable. That is, in a most preferred aspect of the present invention, the structural unit c2 is derived from 2-methoxyethyl acrylate (MEA).

The copolymer according to the present invention essentially includes the structural unit c1 derived from the second monomer and the structural unit c2 derived from the second antithrombogenic monomer, but may include other structural units in addition to these structural units. When the copolymer has another structural unit, examples of the monomer constituting another structural unit include acrylic acid, methacrylic acid, acryloylmorpholine, N,N-dimethylaminoethyl acrylate, N-vinylpyrrolidone, 2-methacryloyloxyethyl phosphorylcholine, 2-methacryloyloxyethyl-D-glycoside, 2-methacryloyloxyethyl-D-mannoside, vinyl methyl ether, 2-hydroxyethyl (meth)acrylate, 4-hydroxybutyl (meth)acrylate, 2-hydroxypropyl (meth)acrylate, 2-hydroxybutyl (meth)acrylate, 6-hydroxyhexyl (meth)acrylate, 1,4-cyclohexanedimethanol mono (meth)acrylate, 1-chloro-2-hydroxypropyl (meth)acrylate, 1,6-hexanediol mono (meth)acrylate, pentaerythritol tri (meth)acrylate, and dipentaerythritol penta (meth)acrylate, neopentyl glycol mono (meth)acrylate, trimethylolpropane di (meth)acrylate, trimethylolethane di (meth)acrylate, 2-hydroxy-3-phenyloxypropyl (meth)acrylate, 4-hydroxycyclohexyl (meth)acrylate, 2-hydroxy-3-phenyloxy (meth)acrylate, 4-hydroxycyclohexyl (meth)acrylate, cyclohexanedimethanol mono (meth)acrylate, adipic acid, glutaric acid, triethylene glycol, and tripropylene glycol. The monomers constituting other structural units may be used singly, or may be used in combination of two or more thereof.

When the copolymer according to the present invention includes other structural units, the content of the other structural units is preferably in a range of more than 0 mol% and 5 mol% or less with respect to all the structural units constituting the copolymer. That is, in the copolymer, when the total of all structural units constituting the copolymer is 100 mol%, the total of the contents of the structural unit c1 and the structural unit c2 is preferably 95 mol% or more (upper limit: less than 100 mol%). More preferably, the copolymer is substantially comprised of the structural unit c1 and the structural unit c2 (the content of other structural units = in a range of more than 0 mol% and less than 5 mol%). In this aspect, more excellent antithrombogenicity can be exhibited. Preferably, the copolymer according to the present invention does not include the other structural unit (content of other structural unit = 0 mol%).

Note that the composition of each structural unit can be measured by a known method. For example, the composition (molar ratio) of the structural unit can be measured by measuring the integral ratio of the intensity of each signal in the ¹H-NMR spectrum of the copolymer solution.

In the polymer according to the present invention, the ratio of the number of structural units b to the number of structural units a is not particularly limited, and the ratio is preferably 30 or more, more preferably in a range of 30 or more and 200 or less, and still more preferably in a range of 30 or more and 150 or less, from the viewpoint of exhibiting sufficient antithrombogenicity. That is, in the medical device according to an embodiment of the present invention, the ratio of the number of structural units b to the number of structural units a in the polymer is 30 or more. By controlling the number of moles of the first antithrombogenic monomer subjected to reaction with (brought into contact with) the base material after the reaction of the first monomer, and the reaction time of the first antithrombogenic monomer with the base material, a polymer in which a predetermined number of structural units b are linked to one structural unit a can be obtained.

The terminal of the polymer chain including the structural unit b is not particularly limited, and may be appropriately defined by the type of monomer used. In an embodiment, the terminal of the polymer chain according to the present invention is a hydrogen atom. When the polymer chain according to the present invention is prepared by a radical polymerization method, the terminal of the polymer can be sealed with a structural unit (functional group) derived from a polymerization initiator. In the latter form (a form sealed with a functional group derived from a polymerization initiator), the terminal of the polymer may be a carboxyl group.

In the polymer according to the present invention, the molar ratio (c1 : c2) of the structural unit c1 derived from the second monomer including an amino group to the structural unit c2 derived from the second antithrombogenic monomer in the structural unit C is not particularly limited. From the viewpoint of improving the reactivity with the structural unit B and the antithrombogenicity, the molar ratio is preferably in a range of 1 : 1 to 1 : 20, more preferably in a range of 1 : 3 to 1 : 18, and still more preferably in a range of 1 : 5 to 1 : 15. That is, in the medical device according to an embodiment of the present invention, the molar ratio (c1 : c2) of the structural unit c1 to the structural unit c2 in the structural unit C is in a range of 1 : 1 to 1 : 20. The molar ratio can be controlled by the blending amount of the second monomer and the second antithrombogenic monomer when the copolymer including the structural unit c1 and the structural unit c2 is synthesized. The composition of each structural unit is substantially equal to the ratio of the charged amount (mol) of each monomer when the copolymer is produced. Note that the molar ratio (c1 : c2) can be measured by using an analytical means such as ¹H-NMR or ¹³C-NMR.

The structure of the copolymer according to the present invention is not particularly limited, and may be any one of a random copolymer, an alternating copolymer, a periodic copolymer, or a block copolymer. Preferably, the copolymer according to the present invention is a random copolymer or a block copolymer. That is, the copolymer according to the present invention is preferably a random copolymer including a segment including the structural unit c1 and a segment including the structural unit c2, or a block copolymer including a block composed of the structural unit c1 and a block composed of the structural unit c2. From the viewpoint of improving the binding property between the structural unit B and the structural unit C (improving the antithrombogenicity), the copolymer according to the present invention is more preferably a random copolymer. The weight average molecular weight of the polymer according to the present invention is in a range of several thousand to several million, and preferably in a range of 10000 to 5000000. In the present description, as the "weight average molecular weight", a value measured by gel permeation chromatography (GPC) using polystyrene as a standard substance and tetrahydrofuran (THF) as a mobile phase is adopted. Note that the molecular weight of the polymer according to the present invention can also be calculated by the type of structural unit and the number of structural units. From the viewpoint of improving the binding property between the structural unit B and the structural unit C (improving the antithrombogenicity), the ratio of the structural unit c1 to the structural unit b in the polymer according to the present invention is preferably in a range of 10 or more and 1000 or less, more preferably in a range of 50 or more and 800 or less, still more preferably in a range of 100 or more and 500 or less, and particularly preferably in a range of 200 or more and 400 or less.

The thickness (dry film thickness) of the antithrombogenic layer is, for example, in a range of 0.1 to 10 um, preferably in a range of 0.5 to 5 µm, and more preferably in a range of about 1 to 3 µm.

### [Method for manufacturing medical device]

Another aspect of the present invention also provides a method for manufacturing the above-described medical device. That is, an embodiment of the present invention is a method for manufacturing the above-described medical device (in the present specification, also simply referred to as "method for manufacturing the medical device according to the present invention"), the manufacturing method including: irradiating at least a part of a surface of the base material with plasma; bringing the first monomer into contact with the base material after the plasma irradiation; bringing the first antithrombogenic monomer into contact with the base material after the first monomer is brought into contact; and bringing the copolymer into contact with the base material after the first antithrombogenic monomer is brought into contact.

Hereinafter, a preferred aspects of each step performed in the method for manufacturing the medical device according to the present invention will be described. Note that the method for manufacturing the medical device according to the present invention is not limited to the following aspect.

### <<Plasma irradiation step (plasma treatment step)>>

In this step, at least a part of the surface of the base material is irradiated with plasma (plasma treatment is performed). As a result, the surface of the base material is oxidized, and as described above, a hydroxyl group (-OH) and a carboxyl group (-COOH) can be generated. Then, by bringing the first monomer into contact with such a surface of the base material, the reactive group (for example, an epoxy group (-C₂H₃O), a glycidyl group (-CH₂(C₂H₃O)), an amino group (-NH₂), or the like) contained in the first monomer may be subjected to a reaction (condensation reaction) with a hydroxyl group (-OH) and a carboxyl group (-COOH) formed on the surface of the base material to form an ether bond (-O-), an amide bond (-C(=O)-NH-), or the like. As a result, since the polymer according to the present invention is firmly fixed on the base material, a medical device that may maintain antithrombogenicity for a long period of time can be manufactured.

A desired plasma treatment can be performed even on a narrow inner surface of an artificial blood vessel, a stent graft, or the like with a small diameter, and therefore the plasma irradiation step is preferably performed by an ionization gas plasma treatment.

Before the surface of the base material is irradiated with ionization gas plasma, the surface of the base material may be cleaned by an appropriate method. That is, before a hydroxyl group (-OH) and a carboxyl group (-COOH) are generated on the surface of the base material by ionization gas plasma irradiation, it is preferable to remove fats and oils, stains, and the like adhering to the surface of the base material. The cleaning treatment method is not particularly limited, and can be performed by cleaning the surface with an appropriate solvent (for example, ultrasonic cleaning, a method for immersing the surface in a cleaning solvent, or a method for pouring a cleaning solvent over the surface).

A pressure condition in the plasma treatment is not particularly limited, and can be either reduced pressure or atmospheric pressure. From the viewpoints that, for example, irradiation with the plasma gas can be performed from a free angle, an apparatus can be reduced in size because a vacuum apparatus is not required, a system configuration can be achieved with a reduced space and cost, and an economically excellent aspect is also obtained, the plasma treatment is preferably performed under atmospheric pressure. By performing irradiation with the plasma gas while rotating a plasma irradiation nozzle around the object to be treated, such as an artificial blood vessel or a stent graft, by one turn, the entire circumference of the object to be treated can be subjected to the plasma treatment without unevenness.

An ionization gas that can be used for the plasma treatment is one or more gases selected from the group consisting of helium, neon, argon, krypton, air, oxygen, carbon dioxide, carbon monoxide, water vapor, nitrogen, hydrogen, and the like. Note that plasma treatment conditions (irradiation time, RF output, overcurrent, gas flow rate, gap between electrodes, and plasma irradiation distance) are not particularly limited, and can be appropriately selected according to a binding property (ease of fixation) between the first monomer and the base material, the type and area of the base material to be used, and the like.

As an example, the irradiation time in the plasma treatment is preferably more than 10 seconds, more preferably in a range of 30 seconds or more and 10 minutes or less, and particularly preferably in a range of 1 to 5 minutes. Under such conditions, a sufficient amount of the first monomer can be bonded (fixed) to the surface of the base material.

As an example, the LF output in the plasma treatment is preferably in a range of 100 to 300 W, and more preferably 200 W.

As an example, the gas flow rate in the plasma treatment is preferably in a range of 5 to 20 sccm, and more preferably 15 sccm.

The temperature of the object to be treated (base material) in the plasma treatment is not particularly limited, and heating or cooling may be performed in addition to room temperature. The plasma treatment is preferably performed at a temperature at which a heating device or a cooling device is unnecessary (for example, in a range of 5 to 35°C) from an economical point of view.

A plasma irradiation apparatus (system) that can be used for the plasma treatment is not particularly limited, and examples thereof include a plasma irradiation apparatus (system) including a plasma generation tube that generates plasma by introducing gas molecules and exciting the gas molecules, and an electrode that excites the gas molecules in the plasma generation tube, in which plasma is emitted from one end of the plasma generation tube, but are not limited to such a configuration (system) at all. Examples thereof include devices adopting a high frequency induction method, a capacity-coupled electrode method, a corona discharge electrode-plasma jet method, a parallel plate type, a remote plasma type, an atmospheric pressure plasma type, a low pressure plasma type, and an ICP type high density plasma type. An ionization gas plasma irradiation apparatus (system) suitable for irradiation of an artificial blood vessel, a stent graft, a covered stent, a catheter, and the like, particularly a plasma irradiation apparatus (system) at atmospheric pressure can be used from among those already commercially available. Specifically, a plasma irradiation apparatus manufactured by Tri-Star Technologies Inc.: DURADYNE (product name or trade name), a plasma irradiation apparatus manufactured by Diener electronic GmbH & Co. KG: PLASMABEAM, Pico Full PC, or the like can be used, but the plasma irradiation apparatus is not limited thereto at all.

After the plasma treatment is performed, the surface of the base material is oxidized by exposing the base material to an oxygen gas-containing atmosphere, and a hydroxyl group (-OH) and a carboxyl group (-COOH) are generated.

### <<First monomer contact step>>

In this step, the first monomer is brought into contact with the base material after the plasma irradiation. As a result, the reactive group (for example, an epoxy group (-C₂H₃O), a glycidyl group (-CH₂(C₂H₃O)), an amino group (-NH₂), or the like) contained in the first monomer may be subjected to a reaction (condensation reaction) with a hydroxyl group (-OH) and a carboxyl group (-COOH) formed on the surface of the base material to form a covalent bond (for example, an ether bond (-O-), an amide bond (-C(=O)-NH-), or the like).

In this step, as a specific method for bringing the first monomer into contact with the base material after the plasma irradiation, either a method for bringing the first monomer as it is into contact with the base material or a method for preparing a first monomer solution in which the first monomer is dissolved in a solvent and then bringing the first monomer solution into contact with the base material may be used. From the viewpoint of forming a more uniform antithrombogenic layer, it is preferable to adopt the latter method for bringing the first monomer solution into contact with the base material.

The solvent used for preparing the first monomer solution is not particularly limited, and is appropriately selected according to the type of the first monomer (and other components when the other components are used). Water (reverse osmosis membrane water (RO water), pure water, deionized water, distilled water, or the like); an alcohol-based solvent such as methanol, ethanol, isopropyl alcohol, or butanol; an organic solvent such as dichloromethane, chloroform, carbon tetrachloride, tetrahydrofuran (THF), dimethyl sulfoxide, N,N-dimethylformamide (DMF), acetone, dioxane, or benzene; and the like are preferably used from the viewpoint of high solubility. These solvents may be used singly or in mixture of two or more types thereof (in a form of a mixed solvent).

The concentration of the first monomer in the first monomer solution is not particularly limited. For example, the concentration of the first monomer in the first monomer solution is preferably in a range of 0.01 to 20 mass%, more preferably in a range of 0.05 to 15 mass%, particularly preferably in a range of 0.1 to 10 mass%, and most preferably in a range of 1 to 5 mass%. When the concentration of the first monomer is within the above range, an antithrombogenic layer to be obtained can sufficiently exhibit the effect of the present invention.

Next, the first monomer solution prepared as described above is brought into contact with the base material after the plasma irradiation. Here, as a specific method of "contact", a conventionally known method such as a method for applying the first monomer solution to the surface of the base material or a method for immersing the base material in the first monomer solution can be appropriately adopted. Among them, it is preferable to use an immersion method that is easy to operate.

Note that, in the case of forming an antithrombogenic layer on a thin and narrow inner surface of an artificial blood vessel, a stent graft, a covered stent, a catheter, or the like, the base material may be immersed in the first monomer solution to reduce the pressure in the system to defoam. The solution can be quickly permeated into the thin and narrow inner surface to promote the binding of the first monomer to the surface of the base material by reducing the pressure to defoam.

Alternatively, the contact by the immersion method may be performed while stirring the first monomer solution. This makes it possible to further promote contact between the first monomer solution and an object to be treated such as an artificial blood vessel or a stent graft. The stirring can be performed using a stirring bar or using a known apparatus such as an orbital shaker, a rotary shaker, or a paint shaker. A stirring condition is not particularly limited. A stirring speed is, for example, in a range of 200 to 600 rpm, and preferably in a range of 300 to 400 rpm from the viewpoint that an object to be treated such as an artificial blood vessel can be more efficiently brought into contact with the first monomer solution. The immersion time (stirring time) is, for example, in a range of 0.5 to 24 hours, and preferably in a range of 1 to 12 hours from a similar viewpoint. Note that the temperature of the first monomer solution at the time of contact by the immersion method may be about room temperature (for example, in a range of 20 to 40°C), but heating (for example, in a range of 40 to 60°C) may be performed.

In the case of forming the antithrombogenic layer only on a part of the base material, the first monomer can be bonded to a desired surface site of the base material by immersing only the part of the base material in the first monomer solution.

When it is difficult to immerse only a part of the base material in the first monomer solution, a surface portion of the base material on which the antithrombogenic layer does not need to be formed is protected (covered or the like) in advance with an appropriate member or material configured to be attached/detached, the base material is immersed in the first monomer solution, and then a protective member (material) of the surface portion of the base material on which the antithrombogenic layer does not need to be formed is removed, whereby the first monomer can be bonded to a desired surface site of the base material. Note that the present invention is not limited to these formation methods, and the first monomer can be bonded by appropriately using a conventionally known method. For example, when it is difficult to immerse only a part of the base material in the first monomer solution, another coating method (for example, a method of coating a first monomer solution to a predetermined surface portion of a medical device using a coating device such as a spray device, a bar coater, a die coater, a reverse coater, a comma coater, a gravure coater, a spray coater, or a doctor knife) may be applied instead of the immersion method. Note that, when both the outer surface and the inner surface of the cylindrical device need to include the antithrombogenic layer due to the structure of the medical device, the immersion method (dipping method) is preferably used because the first monomer can be bonded to both the outer surface and the inner surface at a time.

In this step, if necessary, the base material after being brought into contact with the first monomer may be cleaned. The cleaning treatment method is not particularly limited, and can be performed by cleaning the surface with an appropriate solvent (for example, ultrasonic cleaning, a method for immersing the surface in a cleaning solvent, or a method for pouring a cleaning solvent over the surface). The cleaning time is not particularly limited, but is preferably in a range of 1 to 60 minutes. The cleaning treatment may be repeated a plurality of times (for example, in a range of 2 to 5 times) as necessary.

### <<First antithrombogenic monomer contact step>>

In this step, the first antithrombogenic monomer is brought into contact with the base material after the first monomer is brought into contact. As a result, the ethylenically unsaturated group derived from the first monomer bonded to the base material and the first antithrombogenic monomer are polymerized to form an antithrombogenic layer on the surface of the base material.

In this step, as a specific method for bringing the first antithrombogenic monomer into contact with the base material after the first monomer is brought into contact, either a method for bringing the first antithrombogenic monomer as it is into contact with the base material after the first monomer is brought into contact or a method for preparing a first antithrombogenic monomer solution in which the first antithrombogenic monomer is dissolved in a solvent and then bringing the first antithrombogenic monomer solution into contact with the base material after the first monomer is brought into contact may be used. From the viewpoint of forming a more uniform antithrombogenic layer, it is preferable to adopt the latter method for bringing the first antithrombogenic monomer solution into contact with the base material after the first monomer is brought into contact.

The solvent (polymerization solvent) used for preparation of the first antithrombogenic monomer is not particularly limited as long as it can dissolve the first antithrombogenic monomer and an appropriate polymerization initiator, and examples thereof include one or two or more selected from water (reverse osmosis membrane water (RO water), pure water, deionized water, distilled water, or the like); an alcohol such as ethanol, isopropanol, n-propanol, n-butanol, isobutanol, sec-butanol, t-butanol, ethylene glycol, diethylene glycol, propylene glycol, or dipropylene glycol; an organic solvent such as chloroform, tetrahydrofuran, acetone, dioxane, or benzene; and the like, but are not limited thereto. The concentration of the first antithrombogenic monomer contained in the first antithrombogenic monomer solution is not particularly limited, but by setting the concentration relatively high, the weight average molecular weight of a polymer to be obtained can be increased. As an example, the concentration (total concentration) of the antithrombogenic monomer in the first antithrombogenic monomer solution is preferably 10 mass% or more, and more preferably 15 mass% or more. The upper limit of the monomer concentration (total concentration) is not particularly limited, but is, for example, a saturated concentration or less, and is, for example, 70 mass% or less, and preferably 50 mass% or less.

In this step, by bringing the first antithrombogenic monomer into contact with the base material after the first monomer is brought into contact, the ethylenically unsaturated group derived from the first monomer bonded to the base material and the first antithrombogenic monomer are polymerized (graft-polymerized). A specific method of the polymerization is not particularly limited, and for example, a conventionally known polymerization method such as a radical polymerization method or a polymerization method using a macro initiator can be applied. Examples of the polymerization reaction method include (i) a method in which a first antithrombogenic monomer and an appropriate polymerization initiator are dissolved in a polymerization solvent, and the obtained polymerization reaction liquid is heated to perform a polymerization reaction; and (ii) a method in which a first antithrombogenic monomer is dissolved in a polymerization solvent, the obtained solution is mixed with a separately prepared polymerization initiator solution to prepare a polymerization reaction liquid, and a polymerization reaction is performed. From the viewpoint of simplicity of operation, the method (i) is preferable.

The polymerization reaction liquid may be subjected to a degassing treatment before the polymerization reaction. The degassing treatment can be performed, for example, by bubbling with an inert gas such as nitrogen gas or argon gas for in a range of about 5 minutes to 1 hour.

The polymerization initiator used at this time is not particularly limited, and a known polymerization initiator can be used. Preferably, a radical polymerization initiator is used from the viewpoint of excellent polymerization stability, and specific examples thereof include persulfates such as potassium persulfate (KPS), sodium persulfate, and ammonium persulfate; peroxides such as hydrogen peroxide, t-butyl peroxide, methyl ethyl ketone peroxide, 3-hydroxy-1,1-dimethylbutyl peroxy neodecanoate, α-cumyl peroxy neodecanoate, 1,1,3,3-tetrabutyl peroxy neodecanoate, t-butyl peroxy neodecanoate, t-butyl peroxy neoheptanoate, t-butyl peroxy pivalate, t-amyl peroxy neodecanoate, t-amyl peroxy pivalate, di(2-ethylhexyl)peroxydicarbonate, and di(s-butyl)peroxydicarbonate; and azo compounds such as azobisisobutyronitrile (AIBN), 2,2'-azobis(4-methoxy-2,4-dimethylvaleronitrile), 2,2'-azobis(2,4-dimethylvaleronitrile), 2,2'-azobis[2-(2-imidazoline-2-yl)propane]dihydrochloride, 2,2'-azobis[2-(2-imidazoline-2-yl)propane]disulfate dihydrate, 2,2'-azobis(2-methylpropionamidine)dihydrochloride, 2,2'-azobis[N-(2-carboxyethyl)-2-methylpropionamidine]hydrate, 2,2'-azobis[N-(2-carboxyethyl)-2-methylpropionamidine]tetrahydrate, and 4,4'-azobis(4-cyanovaleric acid). The polymerization initiator may be used singly or in combination of two or more types thereof.

For example, a reducing agent such as sodium sulfite, sodium bisulfite, or ascorbic acid may be used in combination with the radical polymerization initiator, as a redox initiator. The blending amount (addition amount) of the polymerization initiator is preferably in a range of 0.01 to 5 mass%, and more preferably in a range of 0.05 to 3 mass% with respect to 100 mass% of the total of the first antithrombogenic monomer. Alternatively, the blending amount (addition amount) of the polymerization initiator is preferably in a range of 0.01 to 5 mol, and more preferably in a range of 0.05 to 3 mol with respect to 100 mol of the total of the first antithrombogenic monomer. With such a blending amount (addition amount) of the polymerization initiator, the polymerization reaction can be efficiently advanced.

In the case of using the method (ii), the solvent in which the polymerization initiator is dissolved in advance (solvent of the polymerization initiator solution) is not particularly limited as long as it can dissolve the polymerization initiator, but the same solvent as the polymerization solvent can be exemplified. The solvent of the polymerization initiator solution may be the same as or different from the polymerization solvent, but is preferably the same as the polymerization solvent in consideration of ease of polymerization control and the like.

By heating the polymerization reaction liquid prepared as described above, the ethylenically unsaturated group derived from the first monomer bonded to the base material and the first antithrombogenic monomer can be polymerized. Here, as the polymerization method, for example, a known polymerization method such as anionic polymerization or cationic polymerization can be adopted in addition to the above-described radical polymerization, but it is preferable to use radical polymerization in consideration of simplicity of operation.

A polymerization condition is not particularly limited as long as the ethylenically unsaturated group derived from the first monomer bonded to the base material and the first antithrombogenic monomer can be polymerized. As an example, the polymerization temperature is preferably in a range of 30 to 150°C, and more preferably in a range of 40 to 100°C. The polymerization time is preferably in a range of 30 minutes to 30 hours, and more preferably in a range of 1 to 10 hours. Under such conditions, the polymerization reaction can be efficiently advanced.

At the time of polymerization, a chain transfer agent, a polymerization rate adjusting agent, a surfactant, and other additives may be appropriately used as necessary.

The environment in which the polymerization reaction is performed is not particularly limited, and the polymerization reaction can be performed in an air atmosphere, an inert gas atmosphere such as nitrogen gas or argon gas, or the like, but the polymerization reaction is preferably performed in a nitrogen gas atmosphere. During the polymerization reaction, the reaction liquid may be stirred.

In this step, if necessary, the base material after being brought into contact with the first antithrombogenic monomer may be cleaned. The cleaning treatment method is not particularly limited, and can be performed by cleaning the surface with an appropriate solvent (for example, ultrasonic cleaning, a method for immersing the surface in a cleaning solvent, or a method for pouring a cleaning solvent over the surface). The cleaning time is not particularly limited, but is preferably in a range of 1 to 60 minutes. The cleaning treatment may be repeated a plurality of times (for example, in a range of 2 to 5 times) as necessary.

### <<Copolymer contact step>>

In this step, the copolymer is brought into contact with the base material after the first antithrombogenic monomer is brought into contact. As a result, the carboxyl group derived from the antithrombogenic monomer bonded to the base material and the amino group derived from the copolymer are subjected to reaction (dehydration condensation reaction) with each other to form an amide bond (-C(=O)-NH-), whereby the structural unit C derived from the copolymer is fixed.

First, a method for synthesizing a copolymer used in this step will be described. The copolymer can be synthesized by a known polymerization method (a radical polymerization method, a method using macro initiator, or a polycondensation method). For example, when the respective structural units are randomly arranged (the copolymer is a random copolymer), examples of the polymerization method include (i) a method in which the second monomer, the second antithrombogenic monomer, an appropriate polymerization initiator, and a monomer constituting another structural unit added as necessary are dissolved in a polymerization solvent, and the obtained polymerization reaction liquid is heated to perform a polymerization reaction; and (ii) a method in which the second monomer, the second antithrombogenic monomer, and a monomer constituting another structural unit added as necessary are dissolved in a polymerization solvent, the obtained solution is mixed with a separately prepared polymerization initiator solution to prepare a polymerization reaction liquid, and a polymerization reaction is performed. From the viewpoint of simplicity of operation, the method (i) is preferable.

Here, the polymerization solvent is not particularly limited as long as it can dissolve the second monomer, the second antithrombogenic monomer, an appropriate polymerization initiator, and a monomer constituting another structural unit added as necessary, and examples thereof include one or two or more selected from water (reverse osmosis membrane water (RO water), pure water, deionized water, distilled water, or the like); an alcohol such as ethanol, isopropanol, n-propanol, n-butanol, isobutanol, sec-butanol, t-butanol, ethylene glycol, diethylene glycol, propylene glycol, or dipropylene glycol; an organic solvent such as chloroform, tetrahydrofuran, acetone, dioxane, or benzene; and the like, but are not limited thereto. The concentration of the second monomer, the second antithrombogenic monomer, and the monomer constituting another structural unit added as necessary contained in the polymerization reaction liquid is not particularly limited, but by setting the concentration relatively high, the weight average molecular weight of a copolymer to be obtained can be increased. As an example, the concentration (total concentration) of the second monomer, the second antithrombogenic monomer, and the monomer constituting another structural unit added as necessary in the polymerization reaction liquid is preferably 10 mass% or more, and more preferably 15 mass% or more. The upper limit of the monomer concentration (total concentration) is not particularly limited, but is, for example, a saturated concentration or less, and is, for example, 70 mass% or less, and preferably 50 mass% or less.

In the polymerization reaction liquid, the mixing ratio of the second monomer and the second antithrombogenic monomer is preferably adjusted to be the above-described preferable composition (molar ratio).

The polymerization reaction liquid may be subjected to a degassing treatment before the polymerization reaction. The degassing treatment can be performed, for example, by bubbling with an inert gas such as nitrogen gas or argon gas for in a range of about 5 minutes to 1 hour.

The polymerization initiator used at this time is not particularly limited, and a known polymerization initiator can be used. Preferably, a radical polymerization initiator is used from the viewpoint of excellent polymerization stability, and specific examples thereof include persulfates such as potassium persulfate (KPS), sodium persulfate, and ammonium persulfate; peroxides such as hydrogen peroxide, t-butyl peroxide, methyl ethyl ketone peroxide, 3-hydroxy-1,1-dimethylbutyl peroxy neodecanoate, α-cumyl peroxy neodecanoate, 1,1,3,3-tetrabutyl peroxy neodecanoate, t-butyl peroxy neodecanoate, t-butyl peroxy neoheptanoate, t-butyl peroxy pivalate, t-amyl peroxy neodecanoate, t-amyl peroxy pivalate, di(2-ethylhexyl)peroxydicarbonate, and di(s-butyl)peroxydicarbonate; and azo compounds such as azobisisobutyronitrile (AIBN), 2,2'-azobis(4-methoxy-2,4-dimethylvaleronitrile), 2,2'-azobis(2,4-dimethylvaleronitrile), 2,2'-azobis[2-(2-imidazoline-2-yl)propane]dihydrochloride, 2,2'-azobis[2-(2-imidazoline-2-yl)propane]disulfate dihydrate, 2,2'-azobis(2-methylpropionamidine)dihydrochloride, 2,2'-azobis[N-(2-carboxyethyl)-2-methylpropionamidine]hydrate, 2,2'-azobis[N-(2-carboxyethyl)-2-methylpropionamidine]tetrahydrate, and 4,4'-azobis(4-cyanovaleric acid). Among them, the polymerization initiator is preferably an azo compound, more preferably an azo compound having a hydroxyl group or a carboxyl group at the terminal, still more preferably 2,2'-azobis[N-(2-carboxyethyl)-2-methylpropionamidine]hydrate, 2,2'-azobis[N-(2-carboxyethyl)-2-methylpropionamidine]tetrahydrate, or 4,4'-azobis(4-cyanovaleric acid), and particularly preferably 2,2'-azobis[N-(2-carboxyethyl)-2-methylpropionamidine]tetrahydrate, or 4,4'-azobis(4-cyanovaleric acid). The polymerization initiator may be used singly or in combination of two or more types thereof.

For example, a reducing agent such as sodium sulfite, sodium bisulfite, or ascorbic acid may be used in combination with the radical polymerization initiator, as a redox initiator. The blending amount (addition amount) of the polymerization initiator is preferably in a range of 0.01 to 5 mass%, and more preferably in a range of 0.05 to 3 mass% with respect to 100 mass% of the total of the second monomer, the second antithrombogenic monomer, and the monomer constituting another structural unit. Alternatively, the blending amount (addition amount) of the polymerization initiator is preferably in a range of 0.1 to 5 mol, and more preferably in a range of 0.5 to 3 mol with respect to 100 mol of the total of the second monomer, the second antithrombogenic monomer, and the monomer constituting another structural unit. With such a blending amount (addition amount) of the polymerization initiator, a copolymer can be more efficiently produced.

In the case of using the method (ii), the solvent in which the polymerization initiator is dissolved in advance (solvent of the polymerization initiator solution) is not particularly limited as long as it can dissolve the polymerization initiator, but the same solvent as the polymerization solvent can be exemplified. The solvent of the polymerization initiator solution may be the same as or different from the polymerization solvent, but is preferably the same as the polymerization solvent in consideration of ease of polymerization control and the like.

By heating the polymerization reaction liquid prepared as described above, the second monomer, the second antithrombogenic monomer, and the monomer constituting another structural unit can be copolymerized. Here, as the polymerization method, for example, a known polymerization method such as anionic polymerization or cationic polymerization can be adopted in addition to the above-described radical polymerization, but it is preferable to use radical polymerization in consideration of simplicity of operation.

A polymerization condition is not particularly limited as long as it is a condition under which the second monomer, the second antithrombogenic monomer, and a monomer constituting another structural unit can be polymerized. As an example, the polymerization temperature is preferably in a range of 30 to 150°C, and more preferably in a range of 40 to 100°C. The polymerization time is preferably in a range of 30 minutes to 30 hours, and more preferably in a range of 1 to 10 hours. Under such conditions, a copolymer can be more efficiently produced.

At the time of polymerization, a chain transfer agent, a polymerization rate adjusting agent, a surfactant, and other additives may be appropriately used as necessary.

The environment in which the polymerization reaction is performed is not particularly limited, and the polymerization reaction can also be performed in an air atmosphere, an inert gas atmosphere such as nitrogen gas or argon gas, or the like. During the polymerization reaction, the reaction liquid may be stirred.

The copolymer after polymerization is preferably purified by a general purification method such as a reprecipitation method, a dialysis method, an ultrafiltration method, or an extraction method, and particularly, is preferably purified by a reprecipitation method.

The copolymer after purification can be dried by any method such as freeze drying, reduced pressure drying (vacuum drying), spray drying, or heat drying, but from the viewpoint that the influence on the physical properties of the copolymer is small, freeze drying or reduced pressure drying (vacuum drying) is preferable.

For example, when the respective structural units are arranged in a block form (the copolymer is a block copolymer), a living radical polymerization method or a polymerization method using a macro initiator is preferably used. The living radical polymerization method is not particularly limited, but for example, methods described in JP H11-263819 A, JP 2002-145971 A, JP 2006-316169 A, and the like, an atom transfer radical polymerization (ATRP) method, and the like can be applied in the same manner or appropriately modified. In the polymerization method using a macro initiator, for example, a macro initiator including a second monomer and a radical polymerizable group such as a peroxide group is produced, and then the macro initiator and a second antithrombogenic monomer are polymerized in a polymerization solvent, whereby the copolymer can be produced.

Next, the step of bringing the copolymer into contact with the base material after the first antithrombogenic monomer is brought into contact will be described. In this step, a solution containing a copolymer, a solvent, and other components as necessary (also simply referred to as "coating liquid" in the present specification) is prepared, the coating liquid is applied onto a base material, and the carboxyl group derived from the first antithrombogenic monomer and the amino group in the copolymer are subjected to a dehydration condensation reaction to form an amide group.

Examples of other components contained in the coating liquid include a dehydration-condensation agent. In particular, from the viewpoint of promoting the condensation reaction between the carboxyl group (-COOH) of the first antithrombogenic monomer bonded to the base material and the amino group (-NH₂) derived from the copolymer, the coating liquid preferably further contains a dehydration-condensation agent. The dehydration-condensation agent is not particularly limited as long as it is water-soluble and can react with a carboxyl group (-COOH) in an aqueous solution. Specific examples thereof include triazine-based condensing agents such as 4-(4,6-dimethoxy-1,3,5-triazin-2-yl)-4-methylmorpholinium chloride (DMT-MM), water-soluble salts of 1-cyclohexyl-3-(2-morpholinoethyl)carbodiimide, and 1-ethyl-3-(3-dimethylaminopropyl)carbodiimide hydrochloride (WSC).

When the coating liquid further contains a dehydration-condensation agent, the amount of the dehydration-condensation agent in the coating liquid is not particularly limited, but is preferably excessive with respect to the copolymer. For example, the dehydration-condensation agent in the coating liquid is present in an amount in a range of more than 1 mol and 8 mol or less, preferably in a range of 1.5 to 7 mol, and more preferably in a range of 3 to 5 mol, with respect to 1 mol of the copolymer. When the amount of the dehydration-condensation agent is in the above range, the amino group (-NH₂) derived from the copolymer and the carboxyl group (-COOH) derived from the antithrombogenic monomer bonded to the base material can be more efficiently reacted. Note that the dehydration-condensation agent may be used singly or in mixture of two or more types thereof.

The solvent used for preparing the coating liquid is not particularly limited, and is appropriately selected according to the type of the copolymer (and other components when the other components are used). Water (reverse osmosis membrane water (RO water), pure water, deionized water, distilled water, or the like); an alcohol-based solvent such as methanol, ethanol, isopropyl alcohol, or butanol; an organic solvent such as dichloromethane, chloroform, carbon tetrachloride, tetrahydrofuran (THF), dimethyl sulfoxide, N,N-dimethylformamide (DMF), acetone, dioxane, or benzene; and the like are preferably used from the viewpoint of high solubility. These solvents may be used singly or in mixture of two or more types thereof (in a form of a mixed solvent).

The concentration of the copolymer in the coating liquid is not particularly limited. For example, the concentration of the copolymer in the coating liquid is preferably in a range of 0.01 to 20 mass%, more preferably in a range of 0.05 to 15 mass%, and particularly preferably in a range of 0.1 to 10 mass%. When the concentration of the copolymer is within the above range, an antithrombogenic layer to be obtained can sufficiently exhibit the effect of the present invention. The viscosity of the solution falls within an appropriate range, which is preferable in terms of operability (ease of coating, for example) and production efficiency. However, even if it is out of the above range, it can be sufficiently used as long as it does not affect the operation and effect of the present invention.

The method for applying (coating) the coating liquid to the surface of the base layer after bringing the first antithrombogenic monomer into contact with the base material is not particularly limited, and conventionally known methods such as a coating/printing method, an immersion method (dipping method, dip coating method), a spraying method, a spin coating method, a mixed solution impregnation sponge coating method, a bar coating method (for example, the wire bar method), a die coating method, a reverse coating method, a comma coating method, a gravure coating method, and a doctor knife method can be applied. Among them, an immersion method (dipping method, dip coating method), or a bar coating method is preferably used, and an immersion method is more preferably used.

Note that, in the case of forming an antithrombogenic layer on a thin and narrow inner surface of an artificial blood vessel, a stent graft, a covered stent, or the like, the base material may be immersed in the coating liquid to reduce the pressure in the system to defoam. The solution can be quickly permeated into the thin and narrow inner surface to promote the formation of the antithrombogenic layer by reducing the pressure to defoam.

Alternatively, the applying (coating) may be performed while stirring the coating liquid. This makes it possible to further promote contact between the coating liquid and an object to be treated such as an artificial blood vessel. The stirring can be performed using a known apparatus such as an orbital shaker, a rotary shaker, or a paint shaker. A stirring condition is not particularly limited. A stirring speed is, for example, in a range of 100 to 800 rpm, and preferably in a range of 200 to 700 rpm from the viewpoint that an object to be treated such as an artificial blood vessel can be more efficiently brought into contact with the coating liquid. The stirring time is, for example, in a range of 1 to 20 hours from a similar viewpoint. Note that the stirring temperature may be about room temperature (for example, in a range of 20 to 40°C), but heating may be performed as long as the temperature is lower than the boiling point of the solvent used.

The application amount of the coating liquid is preferably selected so that the thickness (dry film thickness) of an antithrombogenic layer to be obtained falls within the above range.

In this step, the antithrombogenic layer may be cleaned if necessary. Here, the cleaning method is not particularly limited, but a method of immersing the antithrombogenic layer in a cleaning solvent, a method of flowing the cleaning solvent onto the antithrombogenic layer, or a combination thereof may be used. The cleaning solvent used at this time is not particularly limited as long as it does not dissolve the antithrombogenic layer, but water (for example, deionized water, distilled water, RO water, filtered water, sterilized water, purified water, or the like) or warm water is preferably used. The temperature of the cleaning water is not particularly limited, but is preferably in a range of 20°C to 80°C. The cleaning time (time for bringing the cleaning solvent into contact with the antithrombogenic layer) is not particularly limited, but is preferably in a range of 1 to 60 minutes. After the cleaning step, a drying step may be further performed. The drying method and the drying conditions (temperature, time, etc.) are not particularly limited, and conventionally known methods can be used.

A medical device excellent in antithrombogenicity is manufactured by the above-described method.

### [Use application of medical device]

The medical device according to the present invention is excellent in antithrombogenicity. Examples of the medical device according to the present invention include implantable prostheses and treating instruments, artificial organs for extracorporeal circulation, catheters, and guide wires. Among them, the medical device is preferably an endovascular indwelling device, and specific examples thereof include an artificial blood vessel, a stent, a stent graft, a covered stent, an artificial valve, a valve clip, and a pump catheter for auxiliary circulation. The medical device according to an embodiment of the present invention is an artificial blood vessel, a stent graft, a covered stent, or a catheter. In addition, the following medical devices are shown. Specific examples thereof include implantable medical devices such as artificial tracheas, artificial skins, and artificial pericardiums, artificial organ systems such as artificial heart systems, artificial lung systems, artificial heart-lung systems, artificial kidney systems, artificial liver systems, and immunoregulation systems, catheters inserted into or indwelled in blood vessels, such as indwelling needles, IVH catheters, catheters for liquid medicine administration, thermodilution catheters, angiographic catheters, vasodilatation catheters, and dilators or introducers, or guide wires, stylets, and the like for these catheters, catheters inserted into or indwelled in biological tissues other than blood vessels, such as various suction catheters including stomach tube catheters, nutrition catheters, tubes for tube feeding (ED), urethral catheters, urine drainage catheters, balloon catheters, and tracheal suction catheters, or drainage catheters.

### Examples

The effects of the present invention will be described with reference to the following Examples and Comparative Examples. Note that the technical scope of the present invention is not limited only to the following Examples. In the following Examples, description "part" or "%" is used, but unless otherwise specified, it represents "part by mass" or "mass%". Unless otherwise specified, each operation was performed at room temperature (25°C).

### <Synthesis of methoxyethyl acrylate (MEA)-aminopropyl methacrylamide (APMA) random copolymer (p(MEA-APMA(14/1)))>

### (1) Preparation

A water bath was filled with tap water and the water surface was covered with polypropylene (PP) balls. Then, this water bath was placed on a magnetic stirrer, and the temperature was set to 80°C.

### (2) Polymerization of p(MEA-APMA)

To a 250 mL three-necked flask, 2.00 g (11 mmol) of N-(3-aminopropyl methacrylamide) hydrochloride (APMA) (Combi-Blocks, Inc.), 20.24 g (156 mmol) of 2-methoxyethyl acrylate (MEA), 0.44 g (1.6 mmol) of 4,4'-azobis(4-cyanovaleric acid) (ACVA) (manufactured by FUJIFILM Wako Pure Chemical Corporation) as a polymerization initiator, 30 mL of 1,4-dioxane, 20 mL of RO water were added. A stirring bar was placed in this three-necked flask, and the mixture was stirred at room temperature (25°C) until all the reagents were dissolved. Nitrogen bubbling was performed from the opening at the end of the three-necked flask for 30 minutes. A cooling tube was erected at the central opening of the three-necked flask, and cooling water was allowed to flow. A thermocouple was inserted from the remaining opening of the three-necked flask to record the solution temperature during the reaction. The three-necked flask was immersed in a water bath set at 80°C, and the reaction was performed for 3 hours. After the reaction for a predetermined time, the three-necked flask was taken out of the water bath and cooled until the temperature returned to room temperature (25°C). As a result, a reaction solution containing p(MEA-APMA) was obtained.

### (3) Purification of p(MEA-APMA)

Hexane and isopropyl alcohol (IPA) were added 250 mL each into a 1 L beaker. The reaction solution obtained in the above 1-2 was poured into this 1 L beaker and stirred for 15 minutes, and then the supernatant was gently removed. The precipitated polymer was dissolved in a 4 : 1 (v/v) tetrahydrofuran (THF)-ethanol mixed solution (THF = 40 mL, ethanol = 10 mL) (polymer solution 1). A 70 : 30 (v/v) hexane/IPA solution (hexane = 350 mL, IPA = 150 mL) was added to the polymer solution 1, the mixture was stirred for 15 minutes to precipitate the polymer, and then the supernatant was gently removed. The precipitated polymer was dissolved again in a 4 : 1 (v/v) tetrahydrofuran (THF)-ethanol mixed solution (THF = 40 mL, ethanol = 10 mL) (polymer solution 2). 500 mL of hexane was added to the polymer solution 2, the mixture was stirred for 20 minutes to precipitate the polymer, and then the supernatant was gently removed. The precipitated polymer was dissolved in a small amount (30 mL) of ethanol and transferred to a poly cup. The poly cup was placed in a draft, the solvent was sufficiently evaporated in the draft, and then vacuumed at room temperature (25°C) for 20 hours to dry the polymer. As a result, an MEA-APMA random copolymer (MEA : APMA = 14 : 1 (molar ratio)) [p(MEA-APMA)] was obtained.

### <Formation of antithrombogenic layer on stent graft made of PET>

### (1) Preparation of stent graft base material made of PET

A stent graft made of polyethylene terephthalate (PET) (length of small diameter portion: 135 mm, diameter: 8 mm) was cut to a length of 2 cm. The cut stent graft made of PET was immersed in ethanol for 1 minute. This operation was repeated three times to perform cleaning, thereby obtaining a stent graft base material made of PET (SG made of PET (plasma-untreated), Comparative Example 1).

### (2) Plasma treatment of stent graft base material made of PET

The cleaned stent graft made of PET was dried in a draft at room temperature overnight, and then set in a plasma irradiation apparatus (Pico Full PC, manufactured by Diener electronic GmbH & Co. KG, low pressure type), and plasma irradiation (argon ionization gas plasma irradiation) was performed for 3 minutes under the conditions of pressure: 0.3 mbar, POWER: 50%, introduction gas: Ar gas 100%, LF output: 200 W, and gas flow rate: 15 sccm. The stent graft made of PET after plasma irradiation was taken out into the atmosphere and left to stand still for 30 minutes. As a result, a stent graft base material made of PET (SG made of PET (after plasma treatment)) whose surface was plasma-treated was obtained.

### (3) Binding of glycidyl methacrylate (GMA)

Into an eggplant flask, 0.435 mL (3.3 mmol) of glycidyl methacrylate (GMA) (manufactured by FUJIFILM Wako Pure Chemical Corporation) and 33 mL of ethanol were placed, and a reagent was dissolved. Nitrogen gas was introduced into the reagent solution in the eggplant flask using a glass tube for 5 minutes to replace the gas in the system with nitrogen gas. The plasma-treated stent graft base material made of PET was placed in the reagent solution, nitrogen gas was further introduced for 5 minutes, and then the opening of the eggplant flask was covered with a rubber stopper. This eggplant flask was placed in a water bath at 37°C and reaction was performed for 4 hours. Thereafter, the stent graft made of PET was taken out, and the unreacted GMA was cleaned with ethanol for 1 hour and dried at room temperature. As a result, a stent graft made of PET to which GMA was bonded (SG-GMA made of PET) was obtained.

### (4) Polymerization of N-methacryloyloxyethyl-N,N-dimethylammonium-α-N-methylcarboxybetaine (CBA)

In a beaker, 5.91 g (27.45 mmol) of N-methacryloyloxyethyl-N,N-dimethylammonium-α-N-methylcarboxybetaine (CBA), 5.7 mg (0.018 mmol) of 2,2'-azobis(4-methoxy-2,4-dimethylvaleronitrile) (V-70, manufactured by FUJIFILM Wako Pure Chemical Corporation), and 30 mL of ethanol were placed, and the mixture was stirred at room temperature until all the reagents were dissolved. The reagent solution in the beaker was transferred to an eggplant flask, nitrogen gas was introduced for 5 minutes using a glass tube to replace the gas in the system with nitrogen gas. The stent graft made of PET to which GMA was bonded (SG-GMA made of PET) was placed in the reagent solution, nitrogen gas was further introduced for 5 minutes, and then the opening of the eggplant flask was covered with a rubber stopper. This eggplant flask was placed in a water bath at 37°C and reaction was performed for 4 hours. Thereafter, the stent graft made of PET was taken out, cleaned by immersion in ethanol in order to remove the unreacted CBA, and dried at room temperature. As a result, a stent graft made of PET and polymerized with CBA via GMA (SG-GMA-pCBA made of PET) was obtained.

### (5) Binding of methoxyethyl acrylate (MEA)-aminopropyl methacrylamide (APMA) copolymer (p(MEA-APMA))

1.248 g (4.5 mmol) of 4-(4,6-dimethoxy-1,3,5-triazin-2-yl)-4-methylmorpholinium chloride (DMT-MM) (dehydration-condensation agent) was dissolved in 1.5 mL of RO water to prepare a DMT-MM solution. 200 mg (1.5 mmol) of the methoxyethyl acrylate (MEA)-aminopropyl methacrylamide (APMA) copolymer (p(MEA-APMA)) synthesized above was dissolved in 20 mL of RO water to prepare a p(MEA-APMA) solution. This p(MEA-APMA) solution was transferred to a 50 mL Falcon (registered trademark, the same applies hereinafter) tube, and a stent graft made of PET and polymerized with CBA via GMA (SG-GMA-pCBA made of PET) was charged. Thereafter, the DMT-MM solution was added dropwise, and the mixture in the Falcon tube was immediately stirred using an orbital shaker. The stirring was performed at a speed of 400 rpm for 3 hours. After stirring, the Falcon tube was removed from the orbital shaker and left to stand still at room temperature overnight to react the primary amino group (-NH₂) of the p(MEA-APMA) copolymer with the carboxyl group (-COOH) of CBA. The stent graft made of PET was removed from the Falcon tube and immersed in 50 mL of RO water for 5 minutes. This operation was repeated three times to perform cleaning, and the stent graft made of PET was taken out of the RO water, left to stand still at room temperature, and dried. As a result, a stent graft made of PET in which an MEA-APMA copolymer was fixed via GMA and CBA polymer chains (SG-GMA-pCBA-p(MEA-APMA) made of PET, Example 1) was obtained.

### <Blood circulation test 1>

For each of SG made of PET (plasma-untreated, Comparative Example 1) and SG-GMA-pCBA-p(MEA-APMA) made of PET of Example 1, which were produced as described above, a blood circulation test was performed by the following method to evaluate antithrombogenicity.

### (1) Preparation of circulation circuit

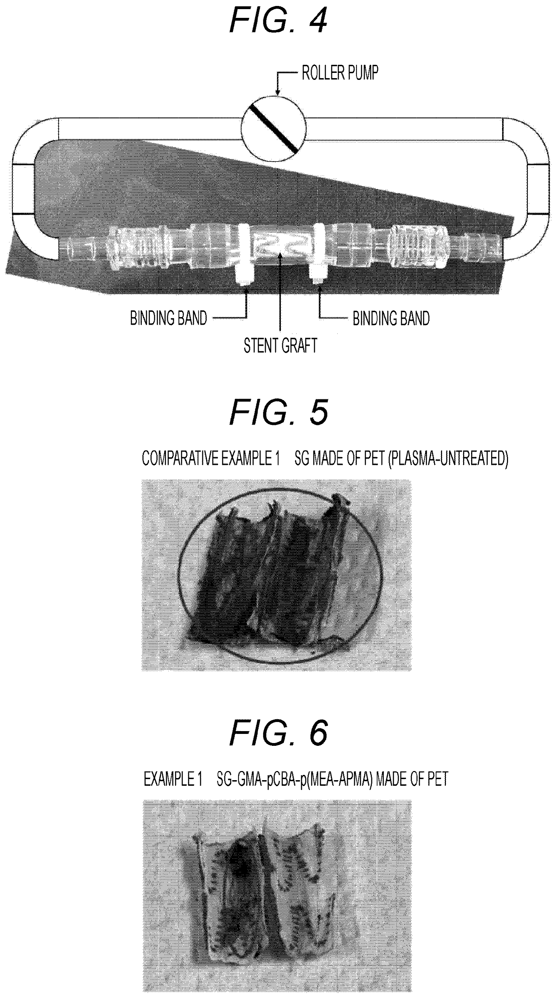

A 0.5 mass% methanol solution of polymethoxyethyl acrylate (PMEA) was passed through a polyvinyl chloride (PVC) tube (SCB04T070 manufactured by SAITAMA CHEMICAL INDUSTRY CO., LTD., size: 6.0 (inner diameter) × 9.0 (outer diameter) mm, 9.5×14.2) and an irregularly-shaped connector (MIMASU CHEMICAL INDUSTRY CO., LTD., CB00X320N manufactured by) to perform antithrombogenic coating. An evaluation sample was inserted into a PVC tube to prepare a circuit (Fig. 4). In order to prevent the position of the stent graft from being displaced during blood circulation, both end portions of the stent graft were narrowed with a binding band from the outside of the tube, and this was used as a blood circulation circuit for the stent graft.

### (2) Obtention of blood

1 mL of heparin (10,000 u/10 mL) and 10 mL of PBS were added in advance to a 1 L blood collection bottle. The pig blood was collected up to a 1000 mL line of the blood collection bottle to obtain blood (1 L) having a heparin concentration of 1 u/mL. After the blood collection, the blood was filtered on the spot using an incorporated filter unit (filter unit: FH-PP47 manufactured by AS ONE Corporation; mesh: HC-58 manufactured by SEFER) and filled in a Terumo separation bag (BB-T020CJ manufactured by Terumo Corporation). Air in the bag was removed as much as possible by crushing the bag. The tube connected to the bag was tied up and sealed, and blood was transported to the laboratory.

### (3) Measurement of activated clotting time (ACT) for blood at experimental initial time point

An operating adapter (TC-MP manufactured by Terumo Corporation) was inserted into the central opening of the bag. The operating adapter was punctured with an indwelling needle (SR-OT1451C manufactured by Terumo Corporation). The catheter was left and the inner needle was pulled out, and a three-way stopcock (TS-TR2K manufactured by Terumo Corporation) was connected to the hub of the catheter. The inside of a 5 mL Terumo syringe was filled with blood, and the inside of the syringe was primed once. Blood was collected into the syringe after priming. A cartridge for ACT measurement (HEIWA BUSSAN CO., LTD., JACT-LR) was inserted into Hemochron (HEIWA BUSSAN CO., LTD., Hemochron Signature Elite), and 50 µL of the collected blood was dropped. The ACT displayed on the screen was recorded. From the above measurement, it was confirmed that ACT was around 200 sec, and blood was used for the experiment.

### (4) Blood circulation test

After filling a 5 mL Terumo syringe with 4 mL of blood, it was connected to the catheter hub of the indwelling needle. The blood circulation circuit was filled with blood through the catheter, and both ends of the circuit were connected (filling rate of blood: 35%). The circuit filled with blood was set in a rotation device installed in an incubator at 40°C. The rotation device was powered on and the blood was circulated at 20 rpm for 2 hours.

### (5) Fixation and observation of sample

After the blood circulation, the rotation device was powered off, and the circulated blood was collected in a pipette tube whose tip was cut. The evaluation sample was removed from the circuit and lightly cleaned with saline. The cleaned sample was immersed in a glutaraldehyde solution (KANTO CHEMICAL CO., INC., 17502-72) diluted to 1 mass% at 4°C for 12 hours or more to perform fixation. Thereafter, the sample was taken out from the glutaraldehyde solution and cleaned with RO water. After drying at room temperature, the appearance was observed. The appearance photographs of samples of Comparative Example 1 and Example 1 are shown in Figs. 5 and 6, respectively.

### (6) Results

As shown in Figs. 5 and 6, it was confirmed that SG-GMA-pCBA-p(MEA-APMA) made of PET of Example 1 significantly suppressed thrombus formation as compared with SG made of PET of Comparative Example 1 (plasma-untreated).

### <Formation of antithrombogenic layer on artificial blood vessel made of ePTFE>

### (1) Preparation of artificial blood vessel base material made of ePTFE

An artificial blood vessel (product name: GORE-TEX (registered trademark) EPTFE Graft II, SGS-054L, manufactured by W. L. Gore & Associates G.K.) made of stretched polyethylene tetrafluoroethylene (ePTFE) was cut into a length of 5 cm. The cut artificial blood vessel made of ePTFE was left to stand still in ethanol to be cleaned, thereby obtaining an artificial blood vessel base material made of ePTFE (ePTFEg (plasma-untreated), Comparative Example 2).

### (2) Plasma treatment of artificial blood vessel base material made of ePTFE

The cleaned artificial blood vessel made of ePTFE was set in a plasma irradiation apparatus (Pico Full PC, manufactured by Diener electronic GmbH & Co. KG, low pressure type), and plasma irradiation (argon ionization gas plasma irradiation) was performed for 3 minutes under the conditions of pressure: 0.3 mbar, POWER: 50%, introduction gas: Ar gas 100%, LF output: 200 W, and gas flow rate: 15 sccm. The artificial blood vessel made of ePTFE after plasma irradiation was taken out into the atmosphere and left to stand still for 30 minutes. As a result, an artificial blood vessel base material made of ePTFE (ePTFEg (after plasma treatment)) whose surface was plasma-treated was obtained.

### (3) Binding of glycidyl methacrylate (GMA)

In a glass bottle, 0.145 mL (1.1 mmol) of glycidyl methacrylate (GMA) (manufactured by FUJIFILM Wako Pure Chemical Corporation) and 11 mL of ethanol were placed, and a reagent was dissolved. The reagent solution in the glass bottle was transferred to a glass tube, nitrogen gas was introduced into the glass tube for 5 minutes to replace the gas in the system with nitrogen gas. The plasma-treated artificial blood vessel base material made of ePTFE was placed in the reagent solution, nitrogen gas was introduced using a glass tube for 5 minutes, and then the glass tube was covered with a rubber stopper. This glass bottle was placed in a water bath at 37°C and reaction was performed for 4 hours. Thereafter, the artificial blood vessel made of ePTFE was taken out, and the unreacted GMA was cleaned with ethanol for 1 hour and dried at room temperature. As a result, an artificial blood vessel made of ePTFE to which GMA was bonded (ePTFEg-GMA) was obtained.

### (4) Polymerization of N-methacryloyloxyethyl-N,N-dimethylammonium-α-N-methylcarboxybetaine (CBA)

In a glass bottle, 1.97 g (9.15 mmol) of N-methacryloyloxyethyl-N,N-dimethylammonium-α-N-methylcarboxybetaine (CBA), 1.9 mg (0.006 mmol) of 2,2'-azobis(4-methoxy-2,4-dimethylvaleronitrile) (V-70, manufactured by FUJIFILM Wako Pure Chemical Corporation), and 10 mL of ethanol were placed, and the mixture was stirred at room temperature until all the reagents were dissolved. The reagent solution in the glass bottle was transferred to a glass tube, nitrogen gas was introduced into the glass tube for 5 minutes to replace the gas in the system with nitrogen gas. The artificial blood vessel made of ePTFE to which GMA was bonded (ePTFEg-GMA) was placed in the reagent solution, nitrogen gas was introduced using a glass tube for 5 minutes, and then the glass tube was covered with a rubber stopper. This glass bottle was placed in a water bath at 37°C and reaction was performed for 4 hours. Thereafter, the artificial blood vessel made of ePTFE was taken out and cleaned by immersion in ethanol to remove the unreacted CBA. As a result, an artificial blood vessel made of ePTFE and polymerized with CBA via GMA (ePTFEg-GMA-pCBA, Comparative Example 3) was obtained.

### (5) Binding of methoxyethyl acrylate (MEA)-aminopropyl methacrylamide (APMA) copolymer (p(MEA-APMA))

1.248 g (4.5 mmol) of 4-(4,6-dimethoxy-1,3,5-triazin-2-yl)-4-methylmorpholinium chloride (DMT-MM) (dehydration-condensation agent) was dissolved in 1.5 mL of RO water to prepare a DMT-MM solution. 200 mg (1.5 mmol) of the methoxyethyl acrylate (MEA)-aminopropyl methacrylamide (APMA) copolymer (p(MEA-APMA)) synthesized above was dissolved in 20 mL of RO water to prepare a p(MEA-APMA) solution. This p(MEA-APMA) solution was transferred to a 50 mL Falcon (registered trademark, the same applies hereinafter) tube, and an artificial blood vessel made of ePTFE and polymerized with CBA via GMA (ePTFEg-GMA-pCBA) was charged. Thereafter, the DMT-MM solution was added dropwise, and the mixture in the Falcon tube was immediately stirred using an orbital shaker. The stirring was performed at a speed of 400 rpm for 3 hours. After stirring, the Falcon tube was removed from the orbital shaker and left to stand still at room temperature overnight to react the primary amino group (-NH₂) of the p(MEA-APMA) copolymer with the carboxyl group (-COOH) of CBA. The artificial blood vessel made of ePTFE was removed from the Falcon tube and immersed in 50 mL of RO water for 5 minutes. This operation was repeated three times to perform cleaning, and the artificial blood vessel made of ePTFE was taken out of the RO water, left to stand still at room temperature, and dried. As a result, an artificial blood vessel made of ePTFE in which an MEA-APMA copolymer was fixed via GMA and CBA polymer chains (ePTFEg-GMA-pCBA-p(MEA-APMA), Example 2) was obtained.

### <Blood circulation test 2>

For each of ePTFEg (plasma-untreated, Comparative Example 2), ePTFEg-GMA-pCBA (Comparative Example 3), and ePTFEg-GMA-pCBA-p(MEA-APMA) (Example 2), which are evaluation samples produced as described above, a blood circulation test was performed by the following method to evaluate antithrombogenicity.

### (1) Preparation of circulation circuit

A 0.5 mass% methanol solution of polymethoxyethyl acrylate (PMEA) was passed through a polyvinyl chloride (PVC) tube (Φ4.7 mm) and a 4.7 mm straight connector to perform antithrombogenic coating. Straight connectors were connected to both ends of the PVC tube, and an evaluation sample was connected between the connectors. To prevent twisting, the outside of the sample was reinforced with a Φ9.5 mm PVC tube. This was used as a blood circulation circuit for an artificial blood vessel.

### (2) Obtention of blood

The same procedure as in "(2) Obtention of blood" in the above <Blood circulation test 1> was performed.

### (3) Measurement of activated clotting time (ACT) for blood at experimental initial time point

The same procedure as in "(3) Measurement of activated clotting time (ACT) for blood at experimental initial time point" in the above <Blood circulation test 1> was performed.

### (4) Blood circulation test

The circuit with a total length of 43 cm was filled with saline and set in a rotation device installed in an incubator at 40°C. The rotation device was powered on and the saline was removed after circulating the saline at 20rpm for 10 minutes. Next, the circuit was filled with 4 mL of blood and set in a rotation device installed in an incubator at 40°C. The rotation device was powered on and the blood was circulated at 20 rpm for 1 hour.

### (5) Fixation and observation of sample

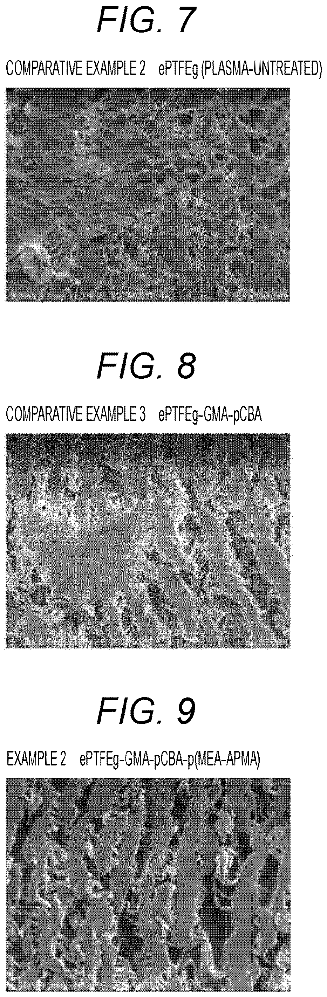

After the blood circulation, the rotation device was powered off, and the circulated blood was collected. The evaluation sample was removed from the circuit and cleaned with saline. The cleaned sample was fixed with a 1 (w/v)% glutaraldehyde PBS solution. The sample was left to stand still overnight at 4°C, and then the sample was cleaned with distilled water. Each sample was dried at room temperature, platinum was deposited, and then observed and photographed (1000 fold) with a scanning electron microscope (SEM). The SEM photographs of samples of Comparative Examples 2 and 3 and Example 2 are shown in Figs. 7 to 9.

### (6) Results

As shown in Fig. 9, in ePTFEg-GMA-pCBA-p(MEA-APMA) of Example 2, thrombus formation on the surface was hardly confirmed. On the other hand, in ePTFEg-GMA-pCBA of Comparative Example 3 shown in Fig. 8, the formation of thrombi was confirmed on a part of the surface. From this, it can be seen that according to the present invention, the antithrombogenicity of the medical device can be further improved as compared with the prior art.

### <Formation of antithrombogenic layer on silicone tube>

### (1) Preparation of silicone tube

A silicone tube (product name: LABORAN (registered trademark) silicone tube manufactured by HAGITEC CO., LTD., outer diameter: 2 mm, inner diameter: 1 mm) was cut to a length of 10 cm. The cut silicone tube was left to stand still in ethanol to be cleaned, thereby obtaining a silicone tube base material (plasma-untreated) (Comparative Example 4).

### (2) Plasma treatment of silicone tube

The cleaned silicone tube was set in a plasma irradiation apparatus (Pico Full PC, manufactured by Diener electronic GmbH & Co. KG, low pressure type), and plasma irradiation (argon ionization gas plasma irradiation) was performed for 3 minutes under the conditions of pressure: 0.3 mbar, POWER: 50%, introduction gas: Ar gas 100%, LF output: 200 W, and gas flow rate: 15 sccm. The silicone tube after plasma irradiation was taken out into the atmosphere and left to stand still for 30 minutes. As a result, a silicone tube base material (silicone tube (after plasma treatment)) whose surface was plasma-treated was obtained.

### (3) Binding of glycidyl methacrylate (GMA)

In a glass bottle, 0.145 mL (1.1 mmol) of glycidyl methacrylate (GMA) (manufactured by FUJIFILM Wako Pure Chemical Corporation) and 11 mL of ethanol were placed, and a reagent was dissolved. The reagent solution in the glass bottle was transferred to a glass tube, nitrogen gas was introduced into the glass tube for 5 minutes to replace the gas in the system with nitrogen gas. The plasma-treated silicone tube base material was placed in the reagent solution, nitrogen gas was introduced using a glass tube for 5 minutes, and then the glass tube was covered with a rubber stopper. This glass bottle was placed in a water bath at 37°C and reaction was performed for 4 hours. Thereafter, the silicone tube was taken out, and the unreacted GMA was cleaned with ethanol for 1 hour and dried at room temperature. As a result, a silicone tube to which GMA was bonded (silicone tube-GMA) was obtained.

### (4) Polymerization of N-methacryloyloxyethyl-N,N-dimethylammonium-α-N-methylcarboxybetaine (CBA)

In a glass bottle, 1.97 g (9.15 mmol) of N-methacryloyloxyethyl-N,N-dimethylammonium-α-N-methylcarboxybetaine (CBA), 1.9 mg (0.006 mmol) of 2,2'-azobis(4-methoxy-2,4-dimethylvaleronitrile) (V-70, manufactured by FUJIFILM Wako Pure Chemical Corporation), and 10 mL of ethanol were placed, and the mixture was stirred at room temperature until all the reagents were dissolved. The reagent solution in the glass bottle was transferred to a glass tube, nitrogen gas was introduced into the glass tube for 5 minutes to replace the gas in the system with nitrogen gas. The silicone tube to which GMA was bonded (silicone tube-GMA) was placed in the reagent solution, nitrogen gas was introduced using a glass tube for 5 minutes, and then the glass tube was covered with a rubber stopper. This glass bottle was placed in a water bath at 37°C and reaction was performed for 4 hours. Thereafter, the silicone tube was taken out and cleaned by immersion in ethanol to remove the unreacted CBA. As a result, a silicone tube polymerized with CBA via GMA (silicone tube-GMA-pCBA) was obtained.

### (5) Binding of methoxyethyl acrylate (MEA)-aminopropyl methacrylamide (APMA) copolymer (p(MEA-APMA))

624 mg (2.25 mmol) of 4-(4,6-dimethoxy-1,3,5-triazin-2-yl)-4-methylmorpholinium chloride (DMT-MM) (dehydration-condensation agent) was dissolved in 5 mL of RO water to prepare a DMT-MM solution. 200 mg (1.5 mmol) of the methoxyethyl acrylate (MEA)-aminopropyl methacrylamide (APMA) copolymer (p(MEA-APMA)) synthesized above was dissolved in 20 mL of RO water to prepare a p(MEA-APMA) solution. This p(MEA-APMA) solution was transferred to a 50 mL Falcon (registered trademark, the same applies hereinafter) tube, and a silicone tube polymerized with CBA via GMA (silicone tube-GMA-pCBA) was charged. Thereafter, the DMT-MM solution was added dropwise, and the mixture in the Falcon tube was immediately stirred using an orbital shaker. The stirring was performed at a speed of 100 rpm for 3 hours. After stirring, the Falcon tube was removed from the orbital shaker and left to stand still at room temperature overnight to react the primary amino group (-NH₂) of the p(MEA-APMA) copolymer with the carboxyl group (-COOH) of CBA. The silicone tube was removed from the Falcon tube and immersed in 50 mL of RO water for 5 minutes. This operation was repeated three times to perform cleaning, and the silicone tube was taken out of the RO water, left to stand still at room temperature, and dried. As a result, a silicone tube in which an MEA-APMA copolymer was fixed via GMA and CBA polymer chains (silicone tube-GMA-pCBA-p(MEA-APMA), Example 3) was obtained.

### <Blood circulation test 3>

For each of the silicone tube (plasma-untreated, Comparative Example 4) and the silicone tube-GMA-pCBA-p(MEA-APMA) (Example 3), which are evaluation samples produced as described above, a blood circulation test was performed by the following method to evaluate antithrombogenicity.

### (1) Preparation of circulation circuit

A 0.5 mass% methanol solution of polymethoxyethyl acrylate (PMEA) was passed through a polyvinyl chloride (PVC) tube (SCB04T070 manufactured by SAITAMA CHEMICAL INDUSTRY CO., LTD., size: 6.0 (inner diameter) × 9.0 (outer diameter) mm, 9.5×14.2) and an irregularly-shaped connector (MIMASU CHEMICAL INDUSTRY CO., LTD., CB00X320N manufactured by) to perform antithrombogenic coating. Both ends of the tube were connected to the connectors and formed in a looped shape. A cut of about 1 cm was made in a position opposite to the connector in a direction parallel to the circuit, and a micropipette tip with a cut tip was inserted into the cut. Next, the evaluation sample was cut into about 10 cm, and then about 6 cm was inserted so as to be attached to the circuit using the lumen of the micropipette tip as a guide. The micropipette tip was removed from the circuit, and a socket of the circuit into which the sample was inserted and an opening outside the circuit of the sample were sealed with an adhesive or the like.

### (2) Obtention of blood

The same procedure as in "(2) Obtention of blood" in the above <Blood circulation test 1> was performed.

### (3) Measurement of activated clotting time (ACT) for blood at experimental initial time point

The same procedure as in "(3) Measurement of activated clotting time (ACT) for blood at experimental initial time point" in the above <Blood circulation test 1> was performed.

### (4) Blood circulation test

The same procedure as in "(4) Blood circulation test" in the above <Blood circulation test 1> was performed.

### (5) Observation of sample

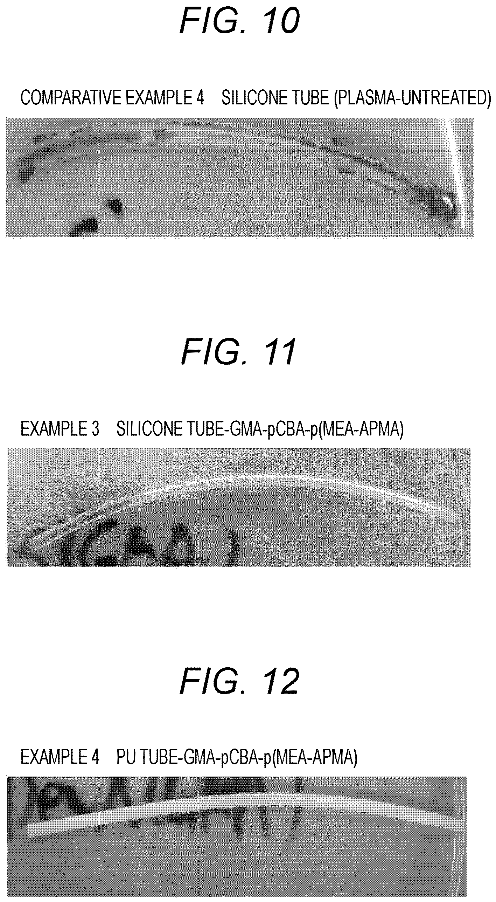

After the blood circulation, the rotation device was powered off, and the circulated blood was collected. The evaluation sample was extracted from the inside of the circuit, cleaned with saline, and then visually observed. The appearance photographs of samples of Comparative Example 4 and Example 3 are shown in Figs. 10 and 11, respectively.

### (6) Results

As shown in Fig. 11, in the silicone tube-GMA-pCBA-p(MEA-APMA) of Example 3, thrombus formation on the surface was hardly confirmed. On the other hand, thrombus formation was confirmed on the surface of the silicone tube of Comparative Example 4 (plasma-untreated) shown in Fig. 10. From this, it can be seen that according to the present invention, the antithrombogenicity of the medical device can be further improved.

### <Formation of antithrombogenic layer on PU tube>

### (1) Preparation of PU tube

A polyurethane (PU) tube (product name: DewX (registered trademark) Eerna manufactured by TERUMO CLINICAL SUPPLY CO., LTD., outer diameter: 1.7 mm, inner diameter: 0.8 mm) was cut to a length of 10 cm. The cut PU tube was left to stand still in ethanol to be cleaned, thereby obtaining a PU tube base material (plasma-untreated).

### (2) Plasma treatment of PU tube

The cleaned PU tube was set in a plasma irradiation apparatus (Pico Full PC, manufactured by Diener electronic GmbH & Co. KG, low pressure type), and plasma irradiation (argon ionization gas plasma irradiation) was performed for 3 minutes under the conditions of pressure: 0.3 mbar, POWER: 50%, introduction gas: Ar gas 100%, LF output: 200 W, and gas flow rate: 15 sccm. The PU tube after plasma irradiation was taken out into the atmosphere and left to stand still for 30 minutes. As a result, a PU tube base material (PU tube (after plasma treatment)) whose surface was plasma-treated was obtained.

### (3) Binding of glycidyl methacrylate (GMA)

In a glass bottle, 0.145 mL (1.1 mmol) of glycidyl methacrylate (GMA) (manufactured by FUJIFILM Wako Pure Chemical Corporation) and 11 mL of ethanol were placed, and a reagent was dissolved. The reagent solution in the glass bottle was transferred to a glass tube, nitrogen gas was introduced into the glass tube for 5 minutes to replace the gas in the system with nitrogen gas. The plasma-treated PU tube base material was placed in the reagent solution, nitrogen gas was introduced using a glass tube for 5 minutes, and then the glass tube was covered with a rubber stopper. This glass bottle was placed in a water bath at 37°C and reaction was performed for 4 hours. Thereafter, the PU tube was taken out, and the unreacted GMA was cleaned with ethanol for 1 hour and dried at room temperature. As a result, a PU tube to which GMA was bonded (PU tube-GMA) was obtained.

### (4) Polymerization of N-methacryloyloxyethyl-N,N-dimethylammonium-α-N-methylcarboxybetaine (CBA)

In a glass bottle, 1.97 g (9.15 mmol) of N-methacryloyloxyethyl-N,N-dimethylammonium-α-N-methylcarboxybetaine (CBA), 1.9 mg (0.006 mmol) of 2,2'-azobis(4-methoxy-2,4-dimethylvaleronitrile) (V-70, manufactured by FUJIFILM Wako Pure Chemical Corporation), and 10 mL of ethanol were placed, and the mixture was stirred at room temperature until all the reagents were dissolved. The reagent solution in the glass bottle was transferred to a glass tube, nitrogen gas was introduced into the glass tube for 5 minutes to replace the gas in the system with nitrogen gas. The PU tube to which GMA was bonded (PU tube-GMA) was placed in the reagent solution, nitrogen gas was introduced using a glass tube for 5 minutes, and then the glass tube was covered with a rubber stopper. This glass bottle was placed in a water bath at 37°C and reaction was performed for 4 hours. Thereafter, the PU tube was taken out and cleaned by immersion in ethanol to remove the unreacted CBA. As a result, a PU tube polymerized with CBA via GMA (PU tube-GMA-pCBA) was obtained.

### (5) Binding of methoxyethyl acrylate (MEA)-aminopropyl methacrylamide (APMA) copolymer (p(MEA-APMA))

624 mg (2.25 mmol) of 4-(4,6-dimethoxy-1,3,5-triazin-2-yl)-4-methylmorpholinium chloride (DMT-MM) (dehydration-condensation agent) was dissolved in 1.5 mL of RO water to prepare a DMT-MM solution. 200 mg (1.5 mmol) of the methoxyethyl acrylate (MEA)-aminopropyl methacrylamide (APMA) copolymer (p(MEA-APMA)) synthesized above was dissolved in 20 mL of RO water to prepare a p(MEA-APMA) solution. This p(MEA-APMA) solution was transferred to a 50 mL Falcon (registered trademark, the same applies hereinafter) tube, and a PU tube polymerized with CBA via GMA (PU tube-GMA-pCBA) was charged. Thereafter, the DMT-MM solution was added dropwise, and the mixture in the Falcon tube was immediately stirred using an orbital shaker. The stirring was performed at a speed of 400 rpm for 3 hours. After stirring, the Falcon tube was removed from the orbital shaker and left to stand still at room temperature overnight to react the primary amino group (-NH₂) of the p(MEA-APMA) copolymer with the carboxyl group (-COOH) of CBA. The PU tube was removed from the Falcon tube and immersed in 50 mL of RO water for 5 minutes. This operation was repeated three times to perform cleaning, and the PU tube was taken out of the RO water, left to stand still at room temperature, and dried. As a result, a PU tube in which an MEA-APMA copolymer was fixed via GMA and CBA polymer chains (PU tube-GMA-pCBA-p(MEA-APMA), Example 4) was obtained.

### <Blood circulation test 4>

The PU tube-GMA-pCBA-p(MEA-APMA) (Example 4) produced above was subjected to a blood circulation test in the same manner as in the above <Blood circulation test 3> to evaluate antithrombogenicity. An appearance photograph of the sample of Example 4 is shown in Fig. 12. As shown in Fig. 12, in the PU tube-GMA-pCBA-p(MEA-APMA) of Example 4, thrombus formation on the surface was hardly confirmed.

The present application is based on Japanese Patent Application No. 2023-116081 filed on July 14, 2023, the entire content of which is incorporated herein by reference.

### Reference Signs List

- 10: Base material
- 10a: Base material core portion
- 10b: Base material surface layer
- 11: Antithrombogenic layer
- 100: Medical device

## Claims

1. A medical device comprising:
a base material;
an antithrombogenic layer including a polymer fixed to the base material, wherein
the polymer includes:
a structural unit a derived from a first monomer bonded to the base material and including a reactive group that reacts with a hydroxyl group or a carboxyl group and an ethylenically unsaturated group;
a structural unit B including a polymer chain including a structural unit b derived from a first antithrombogenic monomer including a carboxyl group and an ethylenically unsaturated group, and including a terminal of the polymer chain bonded to the structural unit a; and
a structural unit C derived from a copolymer including a structural unit c1 derived from a second monomer including an amino group and bonded to the structural unit b and a structural unit c2 derived from a second antithrombogenic monomer.

2. The medical device according to claim 1, wherein the second antithrombogenic monomer is one or two or more selected from a monomer represented by the following Formula (I); 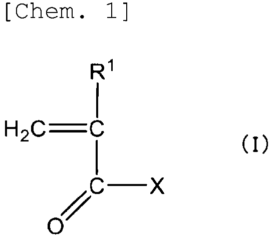 wherein,
R¹ represents a hydrogen atom or a methyl group,
X represents a group represented by formula: -O-(R²O)ₘ-R³, a group represented by formula: -O-(CH₂CHOH)ₙ-CH₂OH, or a tetrahydrofurfuryloxy group, in which each R² independently represents a linear or branched alkylene group having carbon atoms in a range of 1 to 4, R³ represents a linear or branched alkyl group having carbon atoms in a range of 1 to 4, m represents an integer in a range of 1 to 5, and n represents an integer in a range of 1 to 5.

3. The medical device according to claim 2, wherein the second antithrombogenic monomer is one or two or more selected from methoxymethyl acrylate, 2-methoxyethyl acrylate (MEA), 3-methoxypropyl acrylate (MC3A), ethoxymethyl acrylate, ethoxyethyl acrylate (EEA), 2-(2-ethoxyethoxy)ethyl acrylate (EEEA), ethoxypropyl acrylate, ethoxybutyl acrylate, propoxymethyl acrylate, butoxyethyl acrylate, methoxybutyl acrylate, tetrahydrofurfuryl acrylate (THFA), methoxymethyl methacrylate, methoxyethyl methacrylate, ethoxymethyl methacrylate, ethoxyethyl methacrylate, 2-(2-ethoxyethoxy)ethyl methacrylate, propoxymethyl methacrylate, butoxyethyl methacrylate, tetrahydrofurfuryl methacrylate, and glycerol (meth)acrylate.

4. The medical device according to claim 1, wherein the first monomer is one or two or more selected from glycidyl acrylate, glycidyl methacrylate (GMA), 3,4-epoxycyclohexyl methyl acrylate, 3,4-epoxycyclohexyl methyl methacrylate, β-methyl glycidyl acrylate, β-methyl glycidyl methacrylate, and allyl glycidyl ether.

5. The medical device according to claim 1, wherein the first antithrombogenic monomer is one or two or more selected from a monomer represented by the following Formula (II); 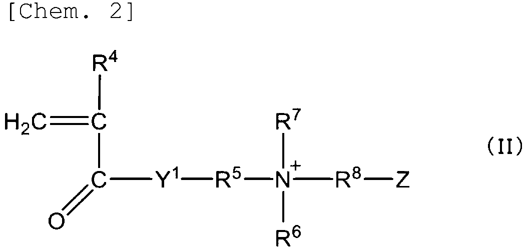 wherein,
R⁴ represents a hydrogen atom or a methyl group,
R⁵ represents a linear or branched alkylene group having carbon atoms in a range of 1 to 6,
R⁶ and R⁷ each independently represent a linear or branched alkyl group having carbon atoms in a range of 1 to 4,
R⁸ represents a linear or branched alkylene group having carbon atoms in a range of 1 to 4,
Z represents -COO⁻, and
Y¹ represents an oxygen atom or -NH-.

6. The medical device according to claim 5, wherein the first antithrombogenic monomer is one or two or more selected from N-(meth)acryloyloxymethyl-N,N-dimethylammonium-α-N-methylcarboxybetaine, N-(meth)acryloyloxyethyl-N,N-dimethylammonium-α-N-methylcarboxybetaine, N-(meth)acryloyloxypropyl-N,N-dimethylammonium-α-N-methylcarboxybetaine, N-(meth)acryloyloxymethyl-N,N-diethylammonium-α-N-methylcarboxybetaine, N-(meth)acryloyloxyethyl-N,N-diethylammonium-α-N-methylcarboxybetaine, and N-(meth)acryloyloxypropyl-N,N-diethylammonium-α-N-methylcarboxybetaine.

7. The medical device according to claim 1, wherein the second monomer is one or two or more selected from a monomer represented by following Formula (III) and a salt thereof; 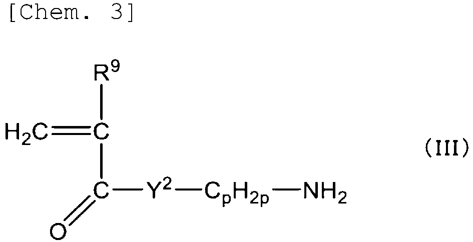 wherein,
R⁹ represents a hydrogen atom or a methyl group,
Y² represents an oxygen atom or -NH-, and
p represents an integer in a range of 1 to 4.

8. The medical device according to claim 7, wherein the second monomer is one or two or more selected from aminomethyl acrylamide, aminomethyl methacrylamide, aminoethyl acrylamide, aminoethyl methacrylamide, aminopropyl acrylamide, aminopropyl methacrylamide (APMA), aminobutyl acrylamide, aminobutyl methacrylamide, aminomethyl acrylate, aminomethyl methacrylate, aminoethyl acrylate, aminoethyl methacrylate, aminopropyl acrylate, aminopropyl methacrylate, aminobutyl acrylate, aminobutyl methacrylate, and salts thereof.

9. The medical device according to claim 1, wherein a molar ratio (c1 : c2) of the structural unit c1 to the structural unit c2 in the structural unit C is in a range of 1 : 1 to 1 : 20.

10. The medical device according to claim 1, wherein the base material includes a plastic base material.

11. The medical device according to claim 10, wherein the plastic base material is a porous body.

12. The medical device according to claim 1, wherein the medical device is an artificial blood vessel, a stent graft, a covered stent, or a catheter.

13. A method for manufacturing the medical device according to claim 1, the manufacturing method comprising:
irradiating at least a part of a surface of the base material with plasma;
bringing the first monomer into contact with the base material after the plasma irradiation;
bringing the first antithrombogenic monomer into contact with the base material after the first monomer is brought into contact; and
bringing the copolymer into contact with the base material after the first antithrombogenic monomer is brought into contact.
